# EUROPÄISCHE PATENTSCHRIFT

(11) **EP 3 559 959 B1**
(45) Veröffentlichungstag und Bekanntmachung des Hinweises auf die Patenterteilung: **04.11.2020**
(21) Anmeldenummer: 18704445.8
(22) Anmeldetag: 25.01.2018
(51) Int. Cl.: H01B 3/40, C08K 5/3445, C08L 63/00, H02K 3/30

(54) **WICKELBANDISOLIERSYSTEM FÜR ELEKTRISCHE MASCHINEN, VERWENDUNG DAZU SOWIE ELEKTRISCHE MASCHINE**
WRAPPING-TAPE INSULATING SYSTEM FOR ELECTRICAL MACHINES, USE THEREFOR, AND ELECTRICAL MACHINE
SYSTÈME D'ISOLATION À RUBAN À ENROULER POUR MACHINES ÉLECTRIQUES, UTILISATION DUDIT SYSTÈME ET MACHINE ÉLECTRIQUE

(30) Priorität: 31.01.2017 DE 102017201498
(43) Veröffentlichungstag der Anmeldung: 30.10.2019
(73) Patentinhaber: Siemens Aktiengesellschaft, 80333 München (DE)
(72) Erfinder: BROCKSCHMIDT, Mario, 45131 Essen (DE); HUBER, Jürgen, 91058 Erlangen (DE); MÜHLBERG, Regina, 06128 Halle (DE); OLBRICH, Irene, 13581 Berlin (DE); ROSSOW, Torsten, 13581 Berlin (DE); SCHIRM, Dieter, 96149 Breitengüßbach (DE); ÜBLER, Matthias, 92289 Ursensollen (DE)
(86) Internationale Anmeldenummer: PCT/EP2018/051814
(87) Internationale Veröffentlichungsnummer: WO 2018/141619

(56) Entgegenhaltungen:
- EP-A1- 0 355 558
- WO-A1-2016/124387
- WO-A1-2016/150764
- WO-A1-2017/012739

## Beschreibung

Die Erfindung betrifft ein Wickelbandisoliersystem für elektrische Maschinen und insbesondere eines, das einen gegenüber dem Stand der Technik vakuumstabileren Bandbeschleuniger hat.

Ein Wickelbandisoliersystem wird beispielsweise zur Isolation von Statorspulen in elektrischen Maschinen eingesetzt, insbesondere wird dabei um einen Leiter oder ein Leiterbündel ein Isolierband gewickelt.

Ein Wickelbandisoliersystem für den Mittel- und Hochspannungsbereich umfasst grundsätzlich ein Isolierband aus dem die Wicklung besteht. Diese Wicklung wird in einem speziellen Vakuumimprägnierverfahren (Vakuumdruckimprägnierung, VPI) mit einem thermisch härtbaren Epoxidharz zur Herstellung des fertigen Wickelbandisoliersystems imprägniert.

Das Isolierband umfasst ein auf einem flexiblen Träger wie Folie oder Glasgewebe aufgebrachtes, flächiges durchschlagfestes anorganisches Material wie Glimmerplättchen und/oder Feinglimmerschichten, das mit dem Träger und miteinander und gegebenenfalls mit einer abschließenden Decklage und oder einer weiteren Lage mittels eines Bandklebers verbunden ist.

Dieser Bandkleber umfasst einen darin gelösten und/oder feinstverteilten Bandbeschleuniger. Der Bandbeschleuniger dient zur Gelierung eines dünnflüssigen Imprägnierharzes, das in der Vakuum-Druck-Imprägnierung (VPI) auf die Wicklungen aufgebracht wird. Nach dem Gelieren bei erhöhter Temperatur werden die imprägnierten Ständerwicklungen beispielsweise in einem so genannten Statorblechpaket thermisch gehärtet.

Damit die Isolierbänder vor der Tränkung lange gelagert werden können, ist im herkömmlichen Isolierband die Bindemittel-Beschleuniger-Mischung so gewählt, dass bei Raumtemperatur praktisch keine Härtung dieser Mischung eintritt.

Ein derartiges Isolierband ist aus der DE 38 24 254 A1 bekannt. Aus der EP 0424376B1 sind entsprechende Bandkleber und Bandbeschleuniger bekannt, die eine ausreichende Lagerstabilität aufweisen.

Die darin offenbarten Bandkleber sind die 1:4-molaren Additionsprodukte aus Bisphenolen, insbesondere des Bisphenol-A, und cycloaliphatischen Epoxidharzen, insbesondere des 3,4-Epoxycyclohexylmethyl-3,4-Epoxycyclohexancarboxylats.

Dieses Bindemittel enthält in seiner theoretischen Molekülstruktur nach quantitativer Umsetzung vermutlich fast ausschließlich cycloaliphatische Oxiranfunktionalitäten neben den durch die Additionsreaktion ebenso geschaffenen Hydroxylgruppen. Weiterhin wird in der EP 0424376B1 offenbart, dass der dazugehörige Bandbeschleuniger bevorzugt ein 1:3-molares Addukt aus Trimethylolpropantriacrylat und N-Ethylpiperazin ist.

Der Bandbeschleuniger und/oder der Bandkleber im Glimmerbandverbund sind insbesondere chemisch so gewählt, dass bei Lagerung unter Raumtemperatur keine vorzeitige und ungewollte Härtung eintritt. Damit wird die Verarbeitbarkeit des Glimmerbandes gewährleistet. Nach erfolgter Imprägnierung der bandkleberhaltigen Statorspulen erfolgt sehr rasch die Gelierung des Epoxidharzes auf Glycidyletherbasis, da endständige Oxiranfunktionalitäten im Zusammenspiel mit Säureanhydriden sehr schneller Polymerisation unterliegen. Durch dieses chemisch abgestimmte Zusammenspiel lässt sich schlussendlich sowohl die gewünschte Lagerstabilität des nicht-imprägnierten Glimmerbandes als auch die rasche Gelierung des imprägnierten Stators realisieren.

Wegen der toxischen Bedenken gegen die uneingeschränkte Verwendung von Phthalsäureanhydrid werden in Zukunft phthalsäureanhydridfreie oder überhaupt anhydridfreie VPI-Harze auf Epoxidbasis Verwendung finden. Dies findet insbesondere in der Echa-Europa-Liste Ausdruck.

Die neuen Härtungskatalysatoren werden auf die anhydridfreien Imprägniermittel abgestimmt sein. Es werden verstärkt anhydridfreie Imprägniermittel, wie aus den älteren Anmeldungen DE 102014219844.5; DE 102014221715.6; DE 102015205328.8, DE 102015202053.3; DE 102015208527.9; DE 102015204885.3, deren Offenbarungsgehalt hiermit zum Inhalt der vorliegenden Beschreibung gemacht wird, bekannt, eingesetzt.

Aus der DE 10 2015 214 872 A1 ist bereits bekannt, dass Stickstoffheterocyclen, wie etwa Imidazole, insbesondere an Stellung 1 und 2 alkylsubstituierte Imidazole, effektive Bandbeschleuniger für phtalsäureanhydridfreie Epoxidharze auf Bisphenol-A- bzw. Bisphenol-F-Diglycidyletherbasis abgeben.

So produziert beispielsweise ein phtalsäureanhydrid- und zudem bindemittelfreier Bisphenol-F-Diglycidylether, der mit N-Ethylpiperazinderivat von Trimethylolpropantriacrylat geliert und für zehn Stunden bei 145°C anionisch-polymerisierend gehärtet wird, lediglich einen Glasübergang von ca. 90°C, wohingegen die phthalsäureanhydridhaltige Mischung mit Bindemittel und Bandbeschleuniger bei identischer Härtung einen Glasübergang von ca. 160°C ausbildet.

Verwendet man dagegen Alkylimidazol, insbesondere 1,2 disubstituierte Diimidazole anstelle des N-Ethylpiperazinderivat von Trimethylolpropantriacrylats, dann steigt die Glasübergangstemperatur auf über 130°C. Deshalb werden in der neuen Generation Bandbeschleuniger, die auf anhydridfreie Epoxidharzmischungen abgestimmt ist, bevorzugt Imidazole, insbesondere Alkylimidazole als Bandbeschleuniger eingesetzt werden.

Nachteilig an den - beispielsweise aus der DE 10 2015 214 872 A1 und/oder den DE 102015213534 A1 und DE 10 2015204885 A1 für diese Verwendung bekannten Alkylimidazolen ist jedoch, dass sich herausgestellt hat, dass die Dampfdrücke der Alkylimidazole relativ hoch liegen, so dass während der im Vakuum-Imprägnierverfahren standardmäßig stattfindenden Evakuierphasen bei erhöhten Temperaturen ein teilweises Austreiben der eingesetzten Imidazole aus dem Glimmerband erfolgt. Dies ist insbesondere deshalb auch sehr nachteilig, weil sie bei erhöhter Temperatur während der Evakuier- und Vortrocknungsphase der zu imprägnierenden Wicklungen aus dem Bandkleber migrieren und zu einer Anreicherung des flüchtigen Alkylimidazols an kälteren Stellen und/oder zu einer Tränkharzkontamination führen können.

Aufgabe der vorliegenden Erfindung ist es daher, ein Isolierband und insbesondere einen Alkylimidazol-Bandbeschleuniger für ein Isolierband zur Verfügung zu stellen, der zusammen mit anhydridfreien VPI-Harzen einsetzbar ist. Zudem ist es Aufgabe der Erfindung, ein Isoliersystem, eine Spule und eine elektrische Maschine mit einem Isolierband anzugeben, das mit einem derartigen anhydridfreien Harz imprägniert ist.

Die Lösung der Aufgabe wird durch den Gegenstand der Erfindung, wie er in der Beschreibung und den Ansprüchen offenbart ist, gegeben.

Dementsprechend ist Gegenstand der vorliegenden Erfindung ein Wickelbandisoliersystem, ein Isolierband mit zumindest einem Bandkleber, darin feinstverteiltem Bandbeschleuniger und ein anhydridfreies Imprägnierharz umfassend, wobei zumindest ein Bandbeschleuniger auf Basis eines an ein Acrylat über die Aminofunktion gebundenen Amino-Imidazol und/oder Aminoalkyl-Imidazols und/oder beliebigen Derivaten davon vorliegt.

Weiterhin ist die Verwendung eines derartigen Wickelbandisoliersystems in elektrischen Maschinen, bevorzugt in rotierenden elektrische Maschinen, besonders bevorzugt in rotierenden elektrische Maschinen im Mittel- und Hochspannungsbereich sowie in elektrischen Schaltanlagen, Mittel- und Hochspannungsanwendungen, Durchführungen, Transformatordurchführungen, Generatordurchführungen und/oder HGÜ-Durchführungen, sowie in entsprechenden Halbzeugen Gegenstand der Erfindung.

Schließlich sind noch elektrischen Maschinen, bevorzugt rotierende elektrische Maschinen, besonders bevorzugt rotierende elektrische Maschinen im Mittel- und Hochspannungsbereich sowie elektrische Schaltanlagen, Mittel- und Hochspannungsanwendungen, Durchführungen, Transformatordurchführungen, Generatordurchführungen und/oder HGÜ-Durchführungen, sowie entsprechende Halbzeuge, die ein derartiges Wickelbandisoliersystem umfassen, Gegenstand der Erfindung.

Allgemeine Erkenntnis der Erfindung ist es, dass die bereits in der DE 102015 214872 beschriebenen 1H-Imidazol-Derivate, beispielsweise das 1H-2-Alkylimidazol durch Addition an eine CC-Doppelbindung von Acrylsäureestern zwar vakuumstabilisiert werden kann, aber die Addition an die CC-Doppelbindung aufgrund der sterischen Hinderung der in 2-Position des Imidazolrings stehenden Seitenkette behindert ist und sich deshalb immer noch "freies", also nicht an das Acrylat gebundenes und deshalb mit hohem Dampfdruck bei bereits niedrigen Temperaturen vorliegendes 1H-2-Alkylimidazol im Bandbeschleuniger befindet, das über die Vakuumimprägnierung ausgetrieben wird.

Durch die Umsetzung mit Amino-Imidazol über die Aminofunktion mittels beispielsweise der Aza-Michael-Addition an die gleichen Acrylate sind Bandbeschleuniger erhältlich, die eine hohe Reaktivität gegenüber dem beim Vakuumimprägnierverfahren eindringenden Imprägnierharz bei gleichzeitiger geringer Reaktivität gegenüber dem Bandkleber zeigen. Entscheidend dabei ist ein äußerst geringer Dampfdruck im Temperaturbereich bis 80°C, so dass unter den Bedingungen der Vakuumdruckimprägnierung nicht die Gefahr der Infizierung der heißhärtenden anhydridfreien Epoxidharzmischung durch Dämpfe beschleunigend und aktivierend wirkender Bestandteile aus der Bandkleber-Beschleuniger-Mischung auftreten kann, wie es mit den bisher aus der DE 10 2015 214872 -deren Offenbarung hiermit zum Inhalt der vorliegenden Beschreibung gemacht wird - bekannten Beschleunigersubstanzen vorkommen kann.

Nach einer vorteilhaften Ausführungsform der Erfindung ist die Bandkleber-Beschleuniger-Mischung so eingestellt, dass sie unter den Bedingungen der Vakuumimprägnierung mit einem anhydridfreien Imprägniermittel mit Gelierzeigen von 1 Stunde bis 15 Stunden bei Imprägniertemperatur abreagieren.

Nach einer Ausführungsform der Erfindung liegt zumindest ein Bandbeschleuniger vor, der ein kovalent über die Aminofunktion an ein höheres Acrylate gebundenes Amino-Imidazol ist.

Als Amino-Imidazol können beispielsweise Aminoalkyl-Imidazol, bevorzugt 1-(Aminoalkyl)-Imidazol, 1H-2-Amino-Imidazol, 1H-2-(Aminoalkyl)-Imidazol, 1H-4-Amino-Imidazol, 1H-4-(Aminoalkyl)-Imidazol, 1H-5-Amino-Imidazol, 1H-5-(Aminoalkyl)-Imidazol sowie die entsprechenden Derivate eingesetzt werden. Beispiele für geeignete Derivate sind: 1-(3-Aminopropyl)imidazol (CAS-Nr. 5036-48-6),
1H-2-Aminoimidazole (CAS-Nr. 7720-39-0),
1H-2-Aminomethylimidazol
1H-2-Aminoethylimidazol
1H-2-Aminopropylimidazol
1H-2-Aminobenzimidazole (CAS-Nr. 934-32-7).

Weitere Beispiele sind Imidazole, die sich von folgenden Strukturen ableiten lassen: 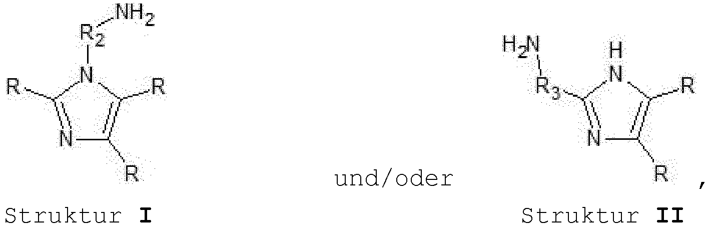 mit
R₂ = (-CH₂-)ₙ wobei n = 1,2,3,...,10
R₃ = (-CH₂-)ₙ wobei n = 1,2, 3,...,10
und R (gleich und/oder ungleich) = Methyl, Ethyl, n-Propyl, iso-Propyl, n-Butyl, iso-Butyl, tert-Butyl, Aryl, insbesondere Phenyl.

Diese werden dann beispielsweise über eine Aza-Michael-Addition an ein höheres Acrylat - insbesondere kovalent - gebunden.

Als höheres Acrylat können beispielsweise Diacrylate, Triacrylate, Tetra-, Penta-, und/oder Hexaacrylate eingesetzt werden, insbesondere

Trimethylolpropantriacrylat (TMPTA, CAS-Nr. 15625-89-5), Trimethylolpropanpropoxylattriacrylat (kein Synonym, CAS-Nr. 53879-54-2),
Pentaerythritoltetraacrylat (PETA, CAS-Nr. 4986-89-4) Dipentaerythritolpentacrylat/Dipentaerythritolhexacrylat (CAS-Nr. 60506-81-2).

Die angestrebten hohen Glasübergangstemperaturen im Werkstoffverbund mit dem phthalsäurefreien Imprägnierharz auf Epoxidharzbasis werden durch die Addition an die höheren Acrylate (Aza-Michael-Addition) erreicht, vgl. Tabelle 1.

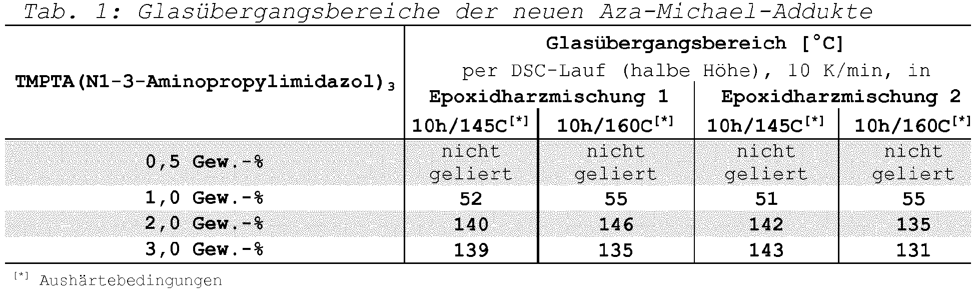

Bei den hier erstmals offenbarten und neuen Bandbeschleunigertypen bilden sich schon ab zwei Gew.-% Anteilen Bandbeschleuniger aus, die zielführende Glasübergangsbereiche im gehärteten Zustand von mehr als 140°C haben, wohingegen bei den Bandbeschleunigern aus dem Stand der Technik derart hohe Glasübergange erst ab einem Bandbeschleunigergehalt von mehr als drei Gew.-% resultieren. Dies spiegelt sich später vorteilhaft in einem geringeren Bandbeschleunigerflächenauftrag wider.

Ebenso vorteilhaft erweisen sich die neuartigen Bandbeschleuniger hinsichtlich der Tendenz zur Gelierung des verwendeten Imprägnierharzes. So ist die Gelzeit um ca. 40-50% gegenüber dem Stand der Technik reduziert, bestimmt wird diese nach einschlägigen und dem Fachmann bekannten Verfahren, z.B. ISO 9396.

Epoxidharzbasis der Imprägnierharzes kann beispielsweise eine oder mehrere Verbindungen, ausgewählt aus der im Folgenden aufgezählten Gruppe, sein:
- Diglycidyletherderivat eines Bisphenol-X, wobei
   X = A, CAS-Nr. 80-05-7,
   X = B, CAS-Nr. 77-40-7,
   X = E, CAS-Nr. 2081-08-5,
   X = F, CAS-Nr. 620-92-8,
   X = AP, CAS-Nr. 1571-75-1
- Glycidylesterderivate, d.h. Diglycidylester aliphatischer, cyclaliphatischer und/oider aromatischer Dicarbonsäuren),
- Glycidyletherderivate von Phenolnovolaken (sog. "Epoxyphenolnovolake")
- Glycidyletherderivate von Cresolnovolaken (sog."Epoxycresolnovolak")
sowie beliebige Gemische der vorgenannten Verbindungen.

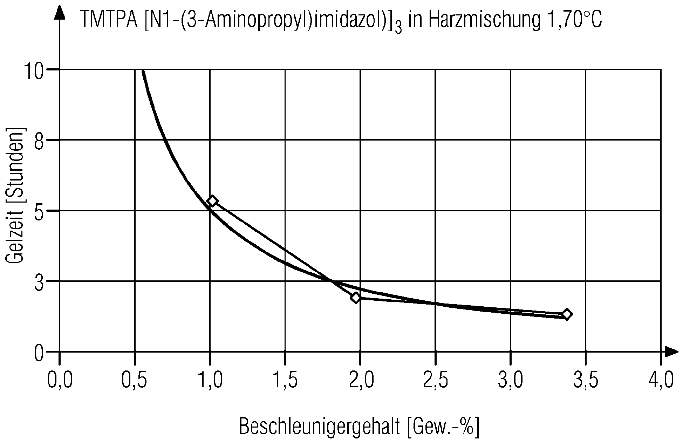

Die in Figur 1 gezeigte Graphik stellt die Gelzeit - Messung gemäß Iso 9396 bei 70°C dar. Es handelt sich um die neue Bandbeschleunigerklasse des Aza-Michael-Addukts aus TMPTA und 3-Aminopropyl-1H-Imidazol in Epoxidharzmischung 1 dar.

Für eine besonders einfache chemische Synthese der hier erstmals vorgestellten Bandbeschleuniger, die über die AminoFunktion an die Acrylate gebunden sind werden insbesondere bei Raumtemperatur flüssige Acrylate mit bei relativ niedrigen Temperaturen schmelzenden Imidazole als Edukte eingesetzt.

Alternativ kann die Synthese in einem geeigneten inerten Lösungsmittel, wie dem Fachmann bekannt ist, erfolgen.

Die Stöchiometrie der Umsetzung ergibt sich - wie dem einschlägigen Fachmann geläufig - aus der Wertigkeit des Acrylats.

Als Bandkleber eignen sich bevorzugt oxirangruppenfreie Bandkleber, da sonst die Lagerstabilität mit den Bandbeschleunigern gemäß der Erfindung fraglich ist. Bevorzugt werden als Bandkleber Copolyester, Diole und/oder höhere Alkohole, sowie beliebige Mischungen davon, eingesetzt. Insbesondere eignen sich dabei die thermoplastischen Verbindungen. Geeignet sind auch lineare, oxirangruppenfreie Verbindungen als Bandkleber.

Beispiele für geeignete Bandkleber sind:
Tricyclomethandimethanol (CAS-Nr. 26896-48-0 bzw. 26160-83-8),
Trimethylolpropan (CAS-Nr. 77-99-6),
dendritische, hydroxy-funktionale Polymere (CAS-Nr. 326794-48-3 bzw. 462113-22-0),
Polycaprolactontriole (CAS-Nr. 37625-56-2),
Polycaprolactontetrole (CAS-Nr. 35484-93-6)
sowie die in der WO 2016/150764 beschriebenen und OxiranGruppenfreien Bandklebermaterialien.

Nachfolgend ist die Struktur III, eine beispielhafte Ausführungsform des Bandbeschleunigers, das Addukte von Amino-Imidazol an Trimethylolpropantriacrylat (TMPTA) gezeigt:

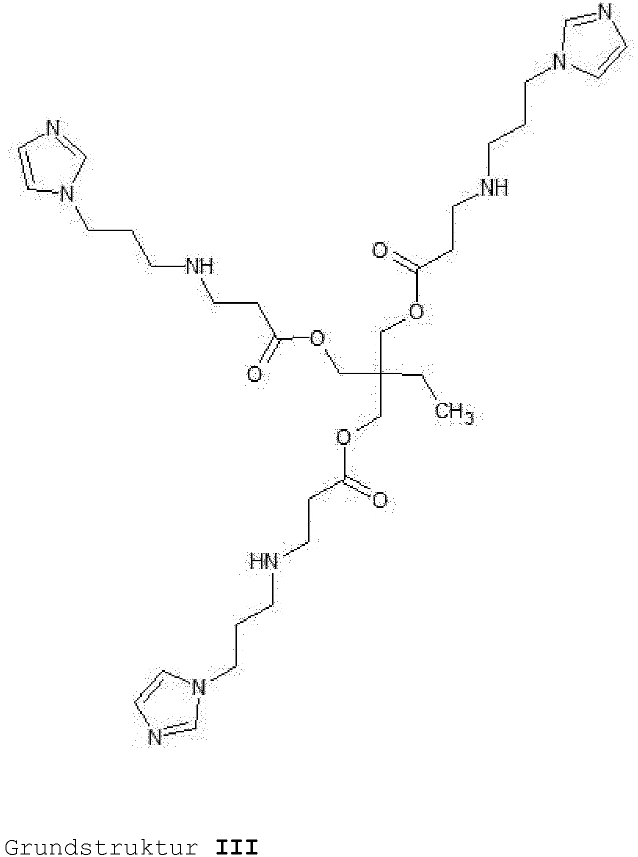

Nachfolgend sind weitere Grundstrukturen IV bis VI gezeigt, die beispielhafte Ausführungsformen eines im Sinne der Erfindung geeigneten Bandbeschleunigers darstellen. Prinzipiell handelt es sich immer um das Addukt aus TMPTA und einem oder mehreren Amino-1H-Imidazol-Derivat(en).

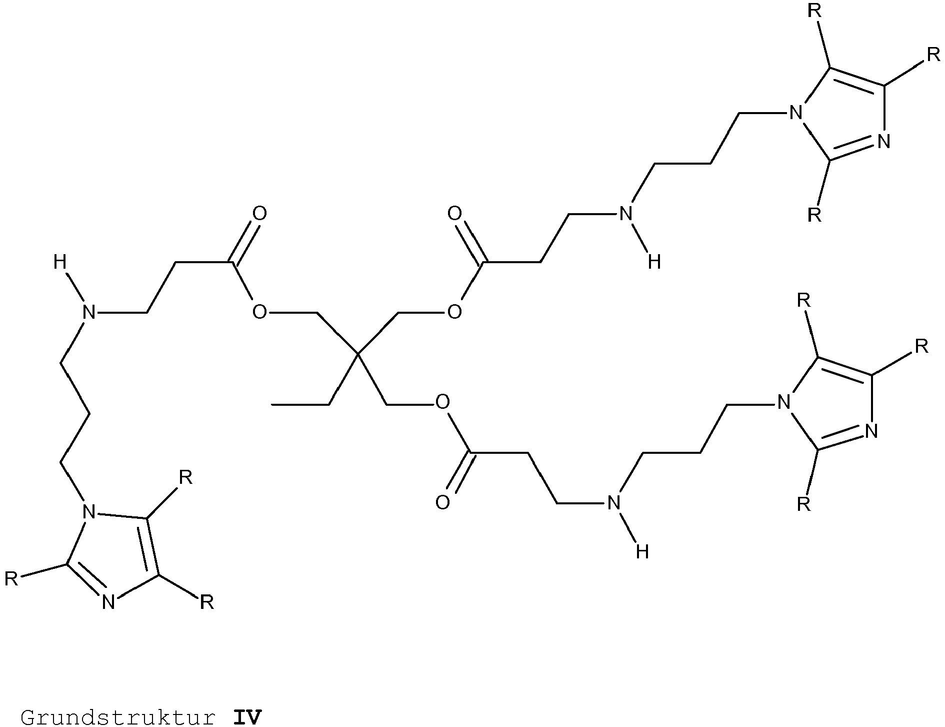

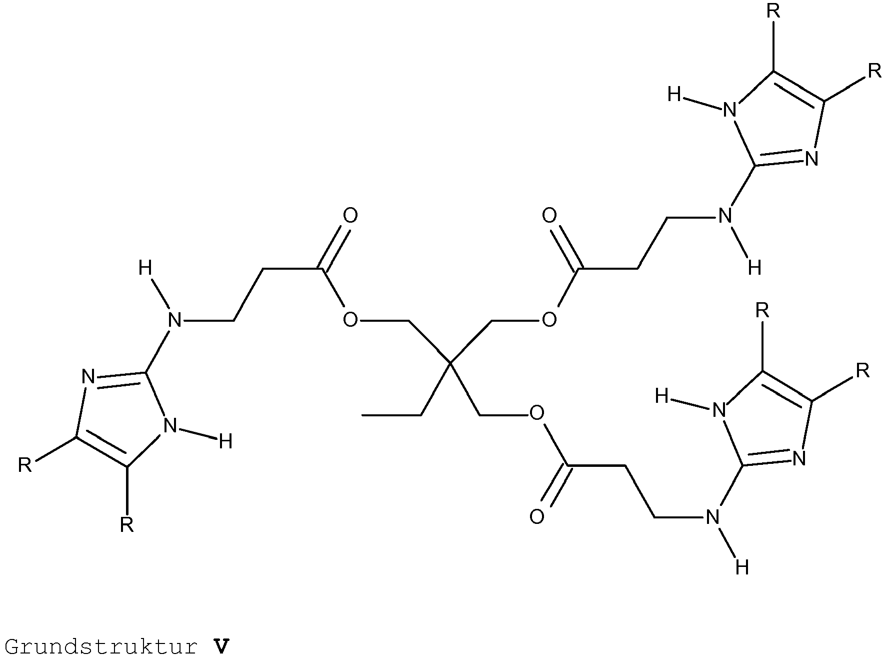

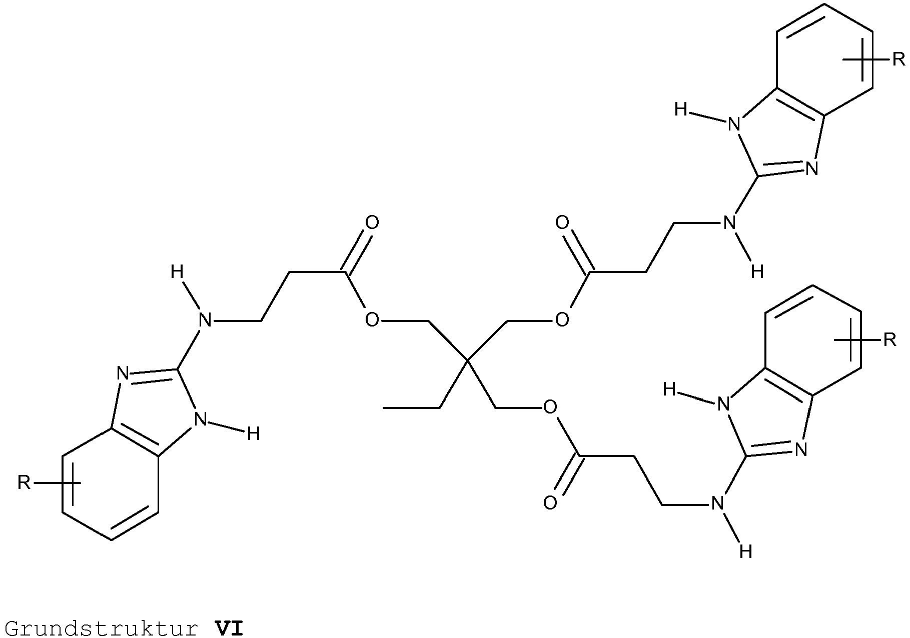

Für die hier gezeigten Strukturformeln **IV** bis **VI** gilt:
**R** = gleich und/oder ungleich und = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl, wobei die Substituenten am Phenylrest wieder gleich oder ungleich und ausgewählt aus der folgenden Gruppe sein können: **R_{phenyl}** = Alkyl (linear und verzweigt), Alkoxy, -F, -Cl, -Br, - J, Aldehyd, Keton, Säureester, Säureamid,
Phosphonsäurederivat und/oder Sulfonsäurederivat

Weitere Ausführungsbeispiele von Addukten von Amino-1H-Imidazolen und Trimethylolpropanpropoxylattriacrylat, die sich gemäß der Erfindung als Bandbeschleuniger eignen sind in den nachfolgenden Strukturen **VII** bis **IX** gezeigt:

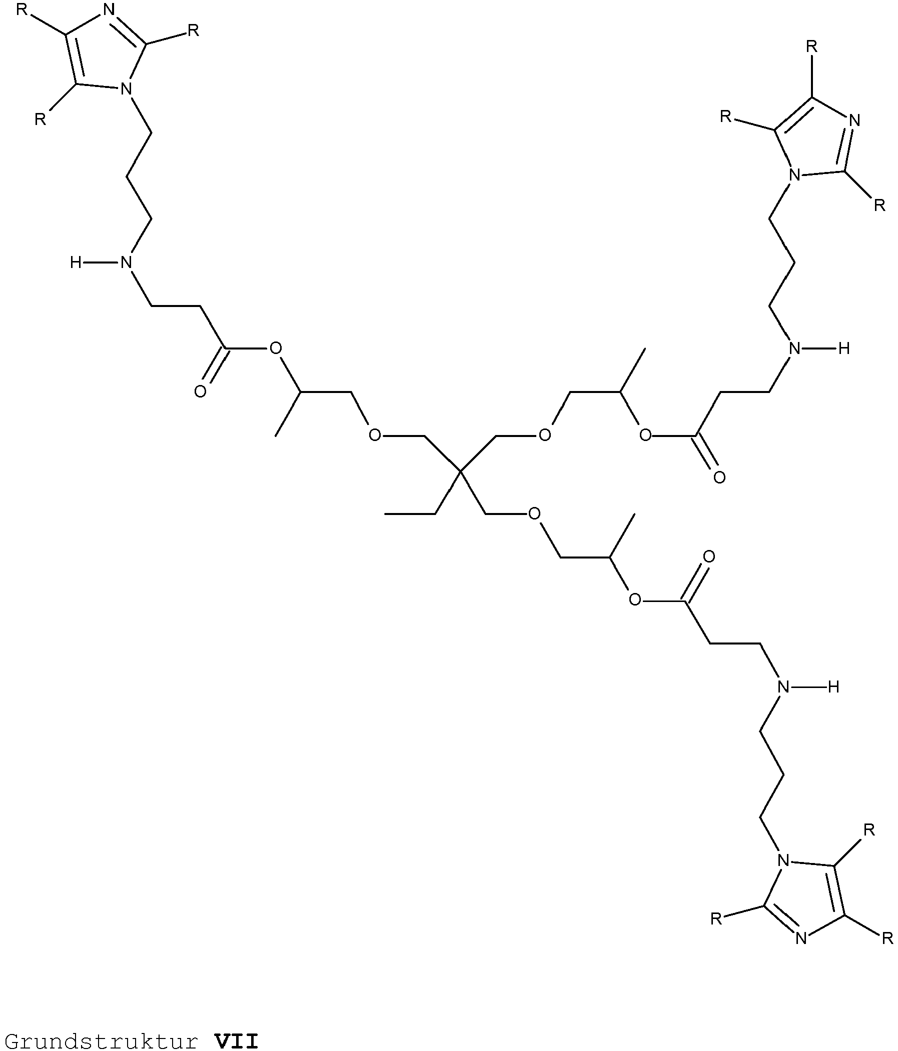

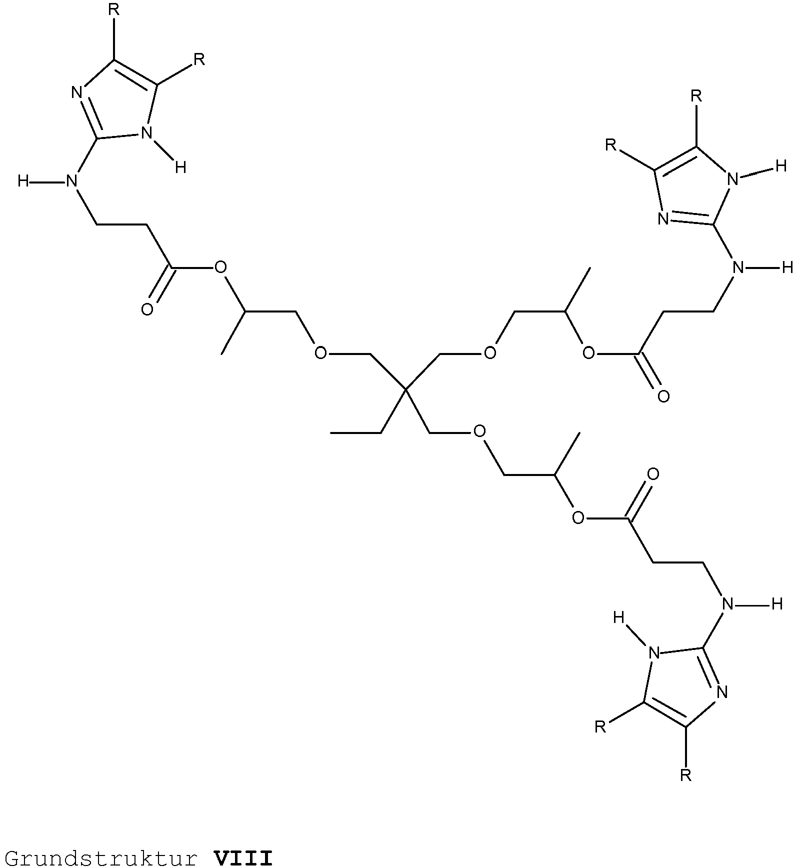

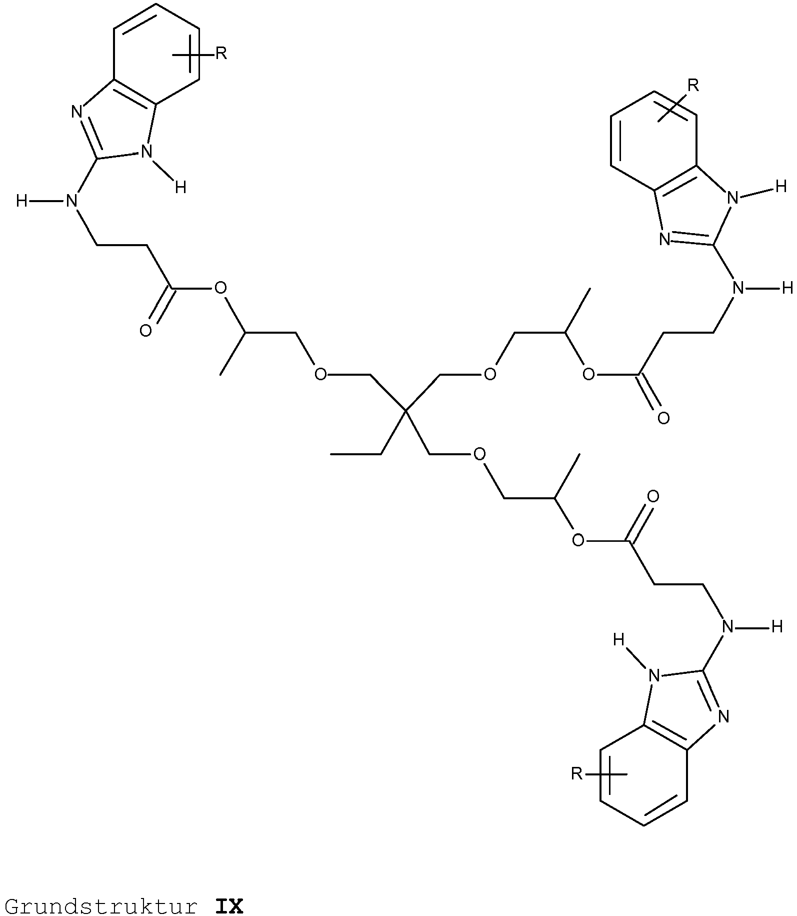

Für die hier gezeigten Strukturformeln **VII** bis **IX** gilt:
**R** = gleich und/oder ungleich und = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl, wobei die Substituenten am Phenylrest wieder gleich oder ungleich und ausgewählt aus der folgenden Gruppe sein können: **R_{phenyl}** = Alkyl (linear und verzweigt), Alkoxy, -F, -Cl, -Br, - J, Aldehyd, Keton, Säureester, Säureamid,
Phosphonsäurederivat und/oder Sulfonsäurederivat.

Weitere Ausführungsbeispiele von Addukten von Amino-1H-Imidazolen und Pentaerythritoltetraacrylat (PETA), die sich gemäß der Erfindung als Bandbeschleuniger eignen, sind in den nachfolgenden Strukturen **X** bis **XII** gezeigt:

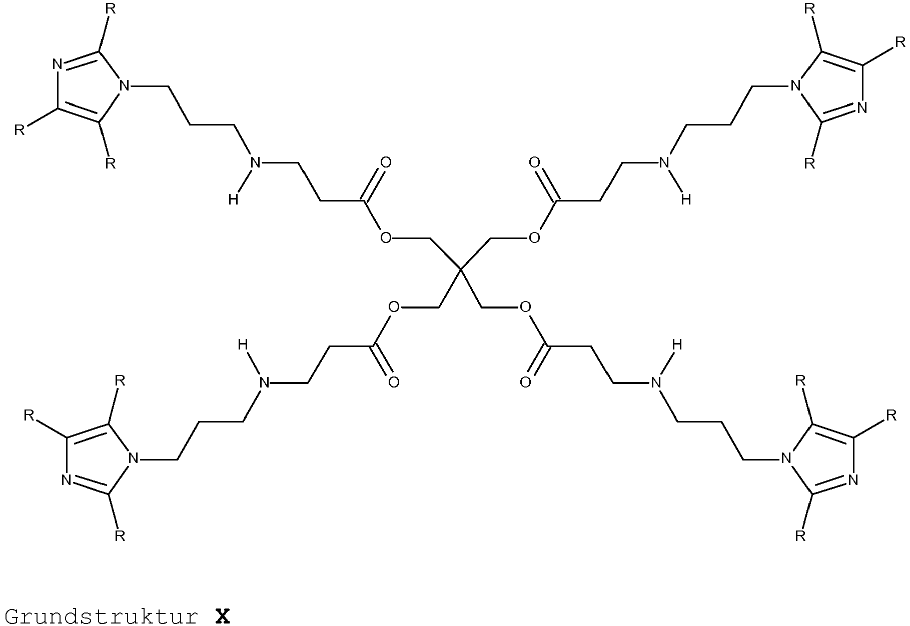

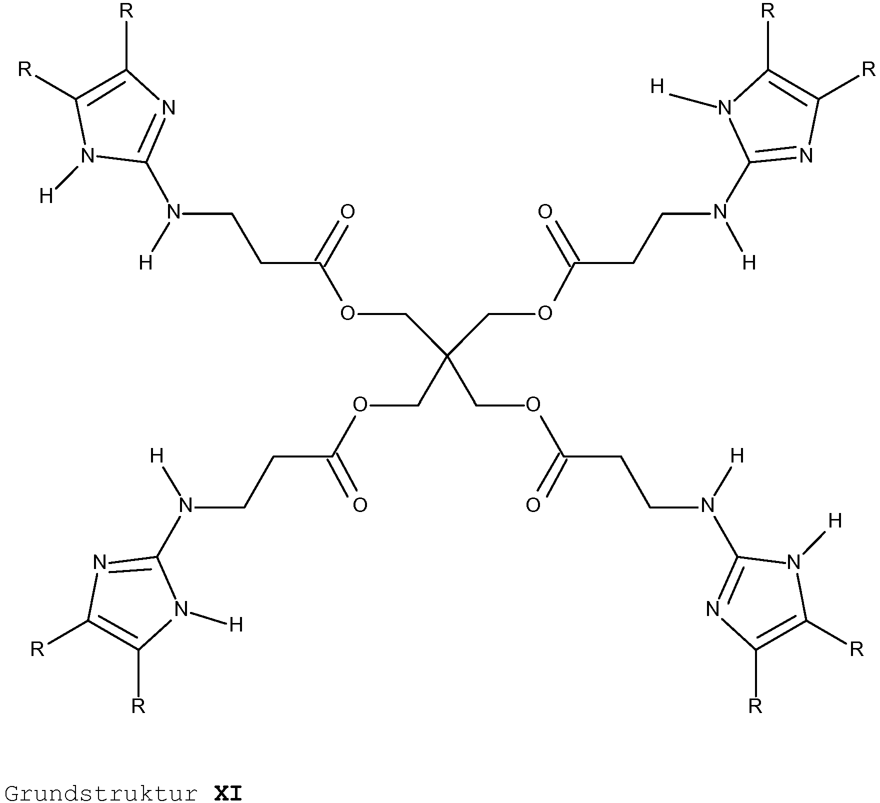

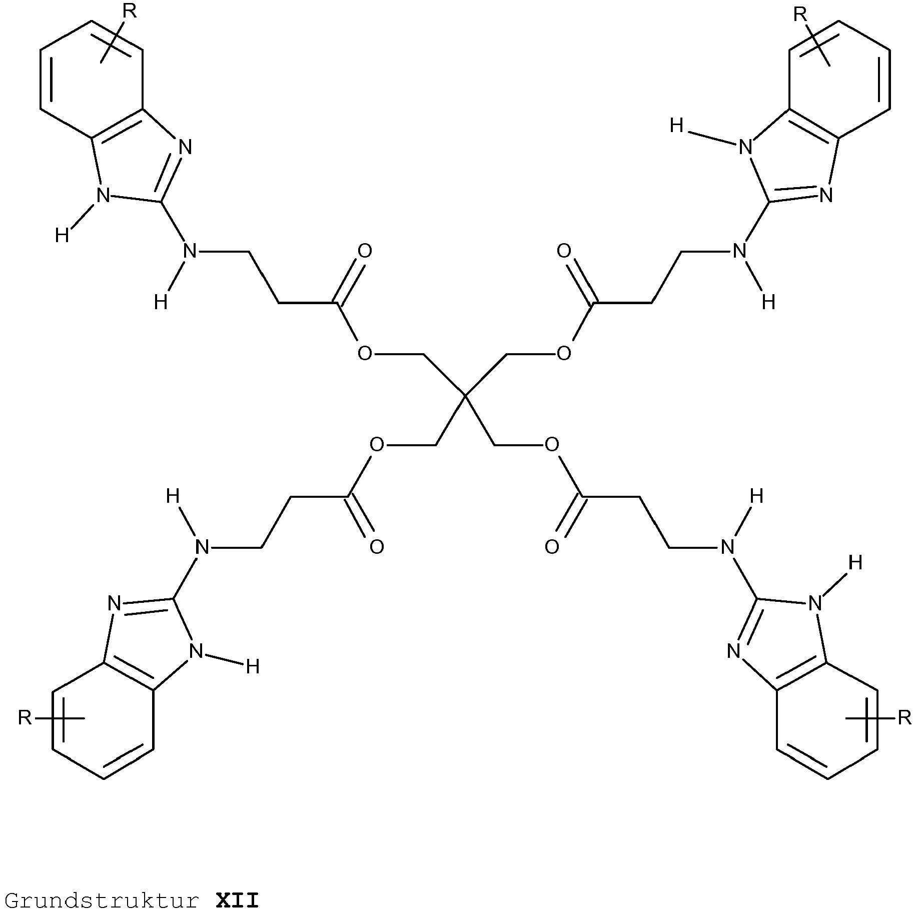

Die Grundstrukturen **X** bis **XII** zeigen Addukte von Amino-Imidazolen an Pentaerythritoltetraacrylat (PETA).

Für die gezeigten Strukturformeln **X** bis **XII** gilt:
**R** gleich und/oder ungleich und ausgewählt aus der Gruppe:
**R** = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl,
wobei die Substituenten am Phenylrest wieder gleich oder ungleich und ausgewählt aus der folgenden Gruppe sein können: **R_{phenyl}** = Alkyl (linear und verzweigt), Alkoxy, -F, -Cl, -Br, - J, Aldehyd, Keton, Säureester, Säureamid, Phosphonsäurederivat und/oder Sulfonsäurederivat.

Weitere Ausführungsbeispiele von Addukten von Amino-1H-Imidazolen und Dipentaerythritolpenta- und/oder hexa-acrylat (DPHA), die sich gemäß der Erfindung als Bandbeschleuniger eignen, sind in der nachfolgenden Struktur **XIII** mit möglichen Resten, wie sie in Strukturen **XIV** bis **XVI** dargestellt sind, gezeigt: 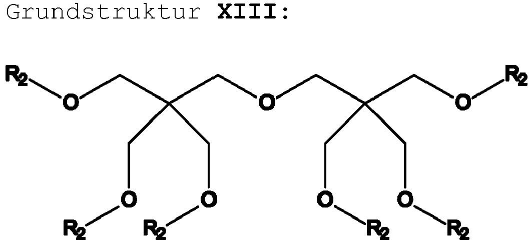 wobei
R₂ = H und/oder 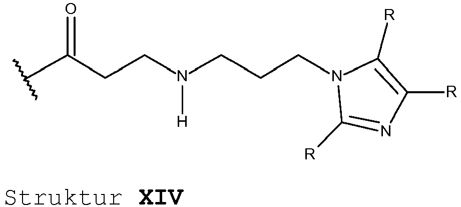 und/oder 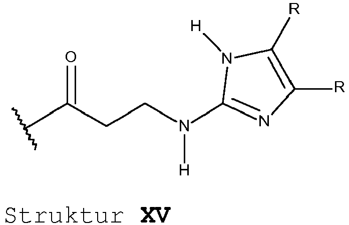 und/oder

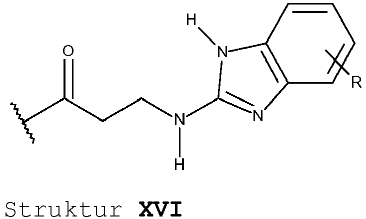

Für die gezeigten Strukturformeln **XIII** bis **XVI** gilt:
**R** = gleich und/oder ungleich und
**R** = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl,
wobei die Substituenten am Phenylrest wieder gleich oder ungleich und ausgewählt aus der folgenden Gruppe sein können: **R_{phenyl}** = Alkyl (linear und verzweigt), Alkoxy, F, Cl, Br, J, Aldehyd, Keton, Säureester, Säureamid, Phosphonsäurederivat und/oder Sulfonsäurederivat
**R₂** wie in den Strukturen **XIV** bis **XVI** angegeben.

Im Vergleich zum Stand der Technik, wie er aus der EP 0424376 B1 bekannt ist, also beispielsweise dem 1:3-molaren Additionsprodukt aus Trimethylolpropantriacrylat ("TMPTA") und N-Ethylpiperazin sind die hier beschriebenen Addukte aus Triacrylaten, insbesondere jedoch auch aus den Tetra- und Penta-/Hexaacrylaten, mit 1-(Aminoalkyl)-Imidazolen, 1H-2-Amino-Imidazolen, 1H-2-(Aminoalkyl)-Imidazolen besonders interessante Verbindungen als Bandbeschleuniger, denn
a) die durch anionische Polymerisation erreichbaren Glasübergangstemperaturen mit phthalsäureanhydridfreien Imprägnierharzen auf Diglycidyletherbasis sind sehr viel höher als bei Verwendung der N-Methylpiperazin- und N-Ethylpiperazinaddukte des TMPTAs
b) 1H-2-Alkyl-Imidazole und (1,2-Alkyl)Imidazole gelieren und härten phthalsäureanhydridfreie Imprägnierharze mit geringeren Gehalten als ein N-Alkylpiperazin-TMPTA-Addukt (Alkyl = beispielsweise Methyl, Ethyl)

Durch die kovalente Anbindung der 1-(Aminoalkyl)-Imidazolen, 1H-2-Amino-Imidazolen, 1H-2-(Aminoalkyl)-Imidazolen über die seitenständig zum Imidazol-Ring befindliche Amino-Gruppe, an das bisher verwendete TMPTA via einer Aza-Michael-Kopplung ist es nunmehr möglich, das für phthalsäureanhydridfreie Epoxidharze ungeeignete N-Ethylpiperazinderivat des TMPTAs gegen eine für die neue Generation an Imprägnierharzen ohne Säureanhydride geeignete Imidazol-TMPTA-Variante zu substituieren.

Insbesondere 1H-2-Amino-Imidazole, 1H-2-(Aminoalkyl)-Imidazole sind hier von Bedeutung, da sie auch nach Addition an z.B. TMPTA noch die 1H-Imidazol-Funktion aufweisen, die für die spätere Härtung des phthalsäureanhydridfreien Epoxidharzes zur Verfügung steht.

In zahlreichen Vorversuchen hat sich gezeigt, dass z.B. 1,2-Dimethylimidazol mit 2 Gew.-% bezogen auf das phthalsäureanhydridfreie Imprägnierharz, insbesondere ein Epoxidharz, hohe Glasübergänge von bis zu 140°C bei sonst identischen Härtungsszenarien liefert, wohingegen der Piperizin-basierte-Bandbeschleuniger nur etwa 90°C als Glasübergang in phthalsäureanhydridfreien Glycidyletherepoxidharzen produziert.

Aufgrund des hohen Dampfdruckes der 1H-2-Alkyl-Imidazole und der (1,2-Alkyl)Imidazole und der hohen Fluidität ist eben ein Dispergieren reiner Imidazole in das Glimmerbandbindemittel mit der späteren Gefahr verbunden, dass die Evakuierphase bei ca. 50°C bis 80°C, bei Unterdruck zu einem Abdampfen bzw. Migrieren des flüchtigen Alkylimidazols führt und dieses sich an kälteren Stellen anreichert. Bei der Harzflutungsphase wird demnach mit einer Harzkontamination gerechnet. Durch eine kovalente Anbindung der Alkylimidazole an ein Acrylat erfolgt eine drastische Verzähung durch Aufbau eines Additions-Bandbeschleunigermoleküls mit einer dynamischen Viskosität bis zu 8 Pa*s oder mehr bei Raumtemperatur. Dadurch wird eine Migration aus dem Glimmerbandbindemittel wirkungsvoll retardiert.

Die hier erstmals offenbarten Bandbeschleuniger sind vorzugsweise vakuumstabil, das heißt beispielsweise, dass sie einen Dampfdruck von kleiner 10⁻³, insbesondere unter 10⁻⁴, bei einer Temperatur von 50°C bis 80°C haben.

Gemäß der Erfindung werden hier erstmals über eine Aminofunktion mit Acrylaten verbundene Imidazole als Bandbeschleuniger oder Härtungskatalysatoren in Wickelbandisoliersystemen vorgestellt. Diese neuartigen Bandbeschleuniger sind inert gegenüber den vorgeschlagenen oxirangruppenfreien Bandklebern und wirken härtungskatalytisch gegenüber dem epoxidbasierten Imprägnierharzen einer Vakuumdruckimprägnierung.

## Patentansprüche

1. Wickelbandisoliersystem, ein Isolierband mit zumindest einem Bandkleber, darin feinstverteiltem Bandbeschleuniger und ein anhydridfreies Imprägnierharz umfassend, wobei zumindest ein Bandbeschleuniger auf Basis eines per Aza-Michael-Addition über die Aminofunktion an ein Acrylat gebundenes Amino-Imidazol und/oder Aminoalkyl-Imidazol und/oder beliebigen Derivaten davon vorliegt, wobei das Wickelbandisoliersystem durch Vakuumdruckimprägnierung herstellbar ist.

2. Wickelbandisoliersystem nach Anspruch 1, wobei zumindest ein Amino-Imidazol ausgewählt ist aus der Gruppe folgender Imidazole: Aminoalkyl-Imidazole allgemein, bevorzugt 1-(Aminoalkyl)-Imidazol, 1H-2-Amino-Imidazol, 1H-2-(Aminoalkyl)-Imidazol, 1H-4-Amino-Imidazol, 1H-4-(Aminoalkyl)-Imidazol, 1H-5-Amino-Imidazol, 1H-5-(Aminoalkyl)-Imidazol sowie die entsprechenden Derivate 1-(3-Aminopropyl)Imidazol (CAS-Nr. 5036-48-6),
1H-2-Aminoimidazole (CAS-Nr. 7720-39-0),
1H-2-Aminomethylimidazol
1H-2-Aminoethylimidazol
1H-2-Aminopropylimidazol
1H-2-Aminobenzimidazole (CAS-Nr. 934-32-7).

3. Wickelbandisoliersystem nach Anspruch 1 oder 2, wobei zumindest ein Amino-Imidazol ausgewählt ist aus der Gruppe der Imidazole, die sich von folgenden Strukturen **I** und **II** ableiten lassen: 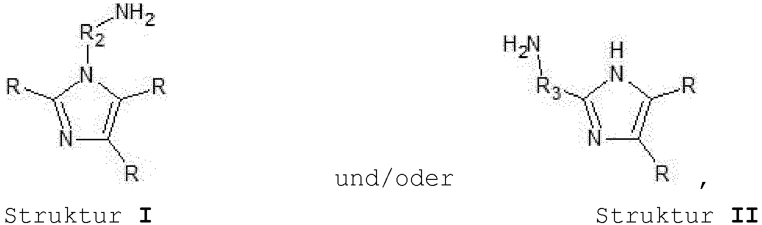 mit
R₂ = (-CH₂-)ₙ wobei n = 1,2,3,...,10
R₃ = (-CH₂-)ₙ wobei n = 1,2, 3,...,10
und R (gleich und/oder ungleich) = Methyl, Ethyl, n-Propyl, iso-Propyl, n-Butyl, iso-Butyl, tert-Butyl, Aryl, insbesondere Phenyl.

4. Wickelbandisoliersystem nach einem der vorstehenden Ansprüche 1 bis 3, wobei zumindest ein Acrylat ein höheres Acrylat ist und insbesondere ausgewählt ist aus der Gruppe folgender Acrylate: Diacrylate, Triacrylate, Tetra-, Penta-, und/oder Hexaacrylate.

5. Wickelbandisoliersystem nach einem der vorstehenden Ansprüche 1 bis 4, wobei zumindest ein höheres Acrylat ausgewählt ist aus der Gruppe folgender Acrylate:
Trimethylolpropantriacrylat (TMPTA, CAS Nr. 15625-89-5), Trimethylolpropanpropoxylattriacrylat (CAS-Nr. 53879-54-2), Pentaerythritoltetraacrylat (PETA, CAS-Nr. 4986-89-4) Dipentaerythritolpentacrylat/Dipentaerythritolhexacrylat (CAS-Nr. 60506-81-2).

6. Wickelbandisoliersystem nach einem der vorstehenden Ansprüche, wobei zumindest ein Bandkleber in Form eines Copolyesters, Diols und/oder höheren Alkohols, sowie beliebige Mischungen davon, vorliegt.

7. Wickelbandisoliersystem nach einem der vorstehenden Ansprüche, wobei der Bandbeschleuniger im Isolierband in einer Menge von weniger als 10 Gew%, bezogen auf die Menge an Imprägnierharz, vorliegt.

8. Wickelbandisoliersystem nach einem der vorstehenden Ansprüche, wobei der Bandkleber im Isolierband in einer Menge im Bereich von 1 bis 30 Gew%, bezogen auf die Menge an Imprägnierharz, vorliegt.

9. Wickelbandisoliersystem nach einem der vorstehenden Ansprüche, wobei zumindest ein Bandbeschleuniger in Form einer Verbindung, mit folgender Struktur **III** vorliegt.

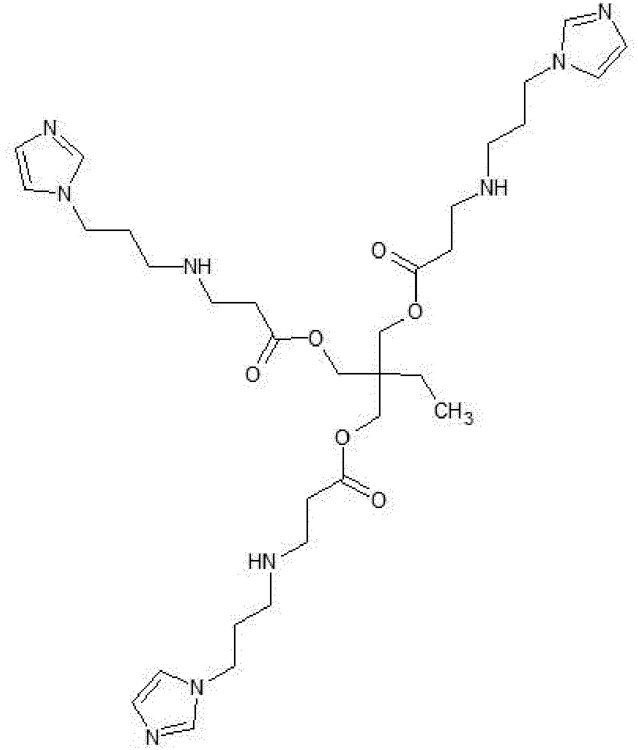

10. Wickelbandisoliersystem nach einem der vorstehenden Ansprüche, wobei zumindest ein Bandbeschleuniger in Form einer Verbindung, deren Struktur abgeleitet ist von zumindest einer der folgenden Grundstrukturen **IV** bis **VI,** enthalten ist: 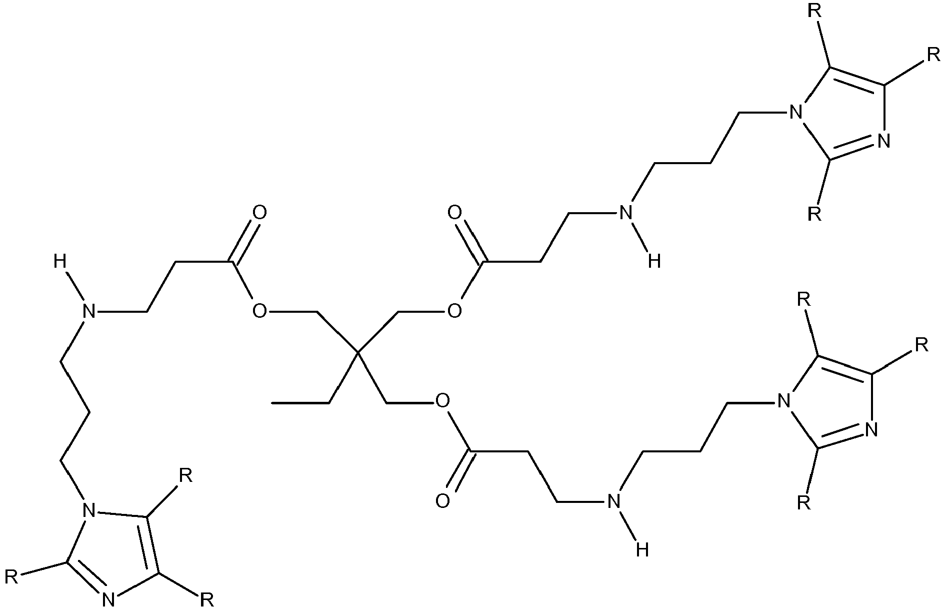 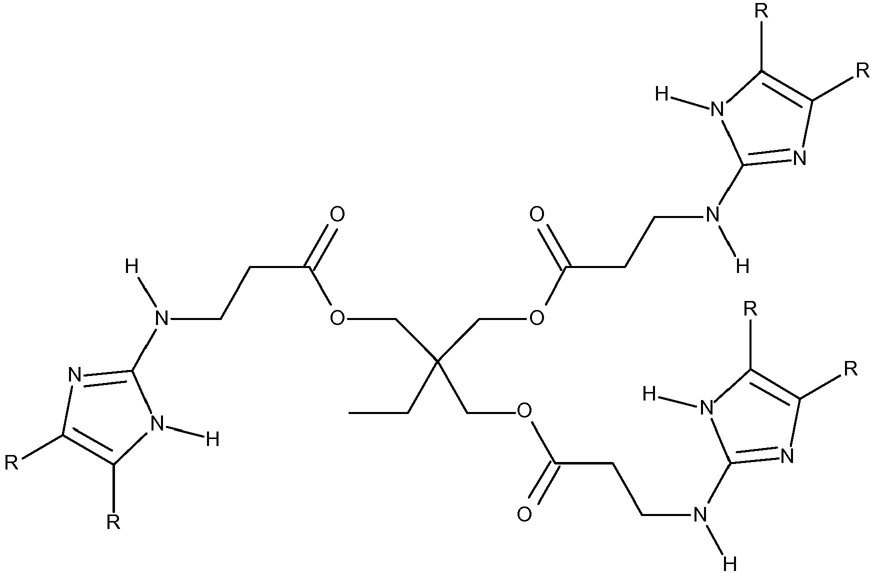 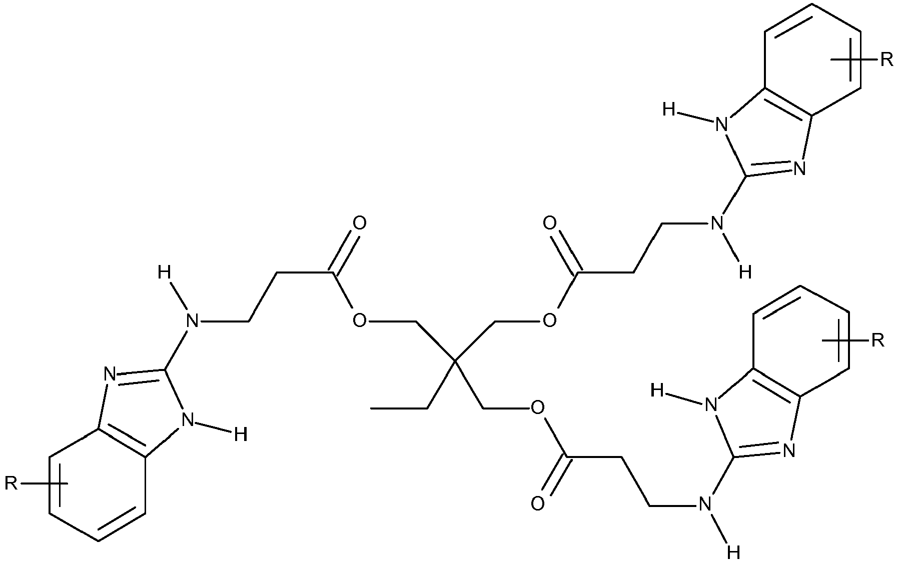 wobei für die hier gezeigten Strukturformeln **IV** bis **VI** gilt: **R** = gleich und/oder ungleich und = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl, wobei die Substituenten am Phenylrest wieder gleich oder ungleich und ausgewählt aus der folgenden Gruppe sein können: **R_{phenyl}** = Alkyl (linear und verzweigt), Alkoxy, -F, -Cl, -Br, - J, Aldehyd, Keton, Säureester, Säureamid, Phosphonsäurederivat und/oder Sulfonsäurederivat.

11. Wickelbandisoliersystem nach einem der vorstehenden Ansprüche, wobei zumindest ein Bandbeschleuniger in Form einer Verbindung, deren Struktur abgeleitet ist von zumindest einer der folgenden Grundstrukturen **VII** bis **IX,** enthalten ist: 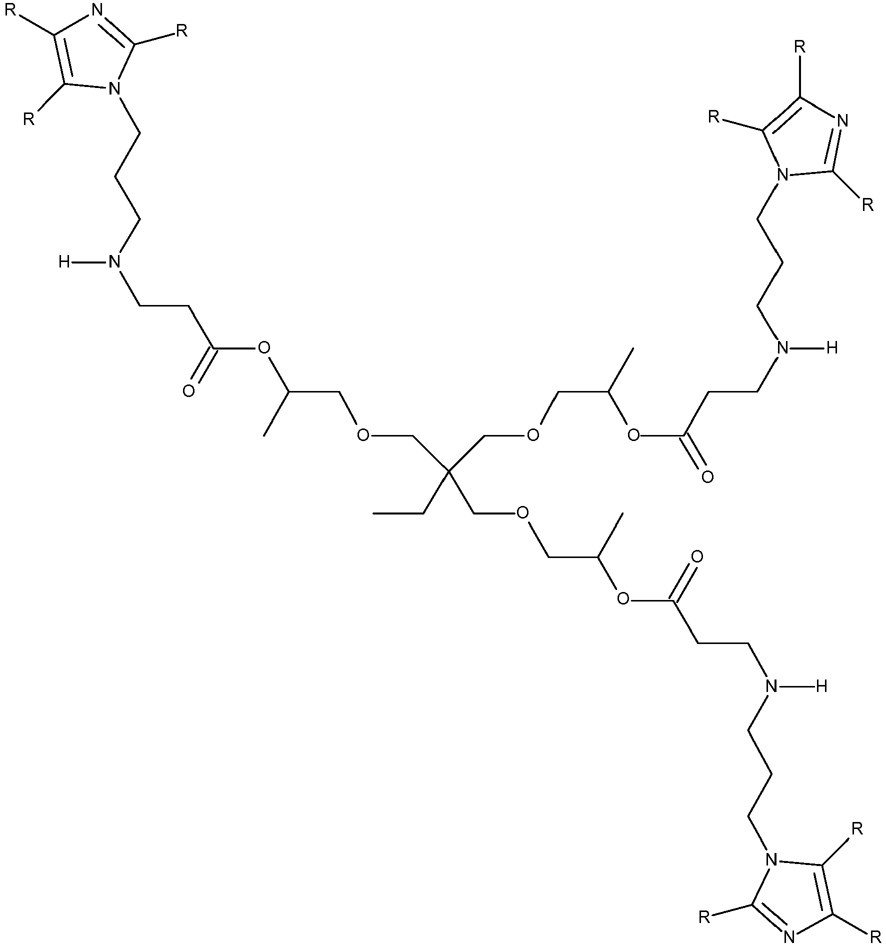 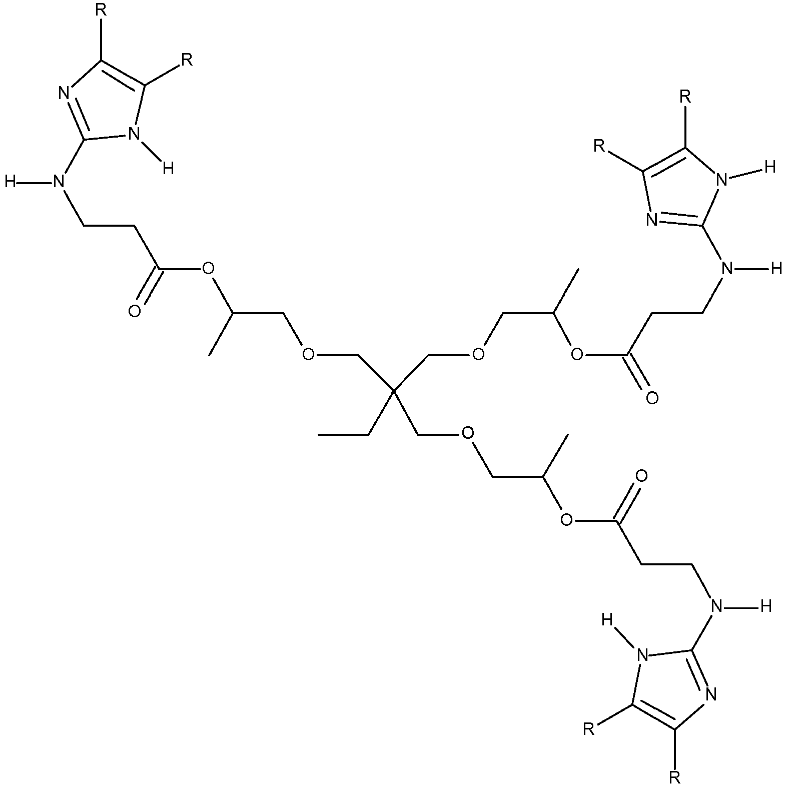 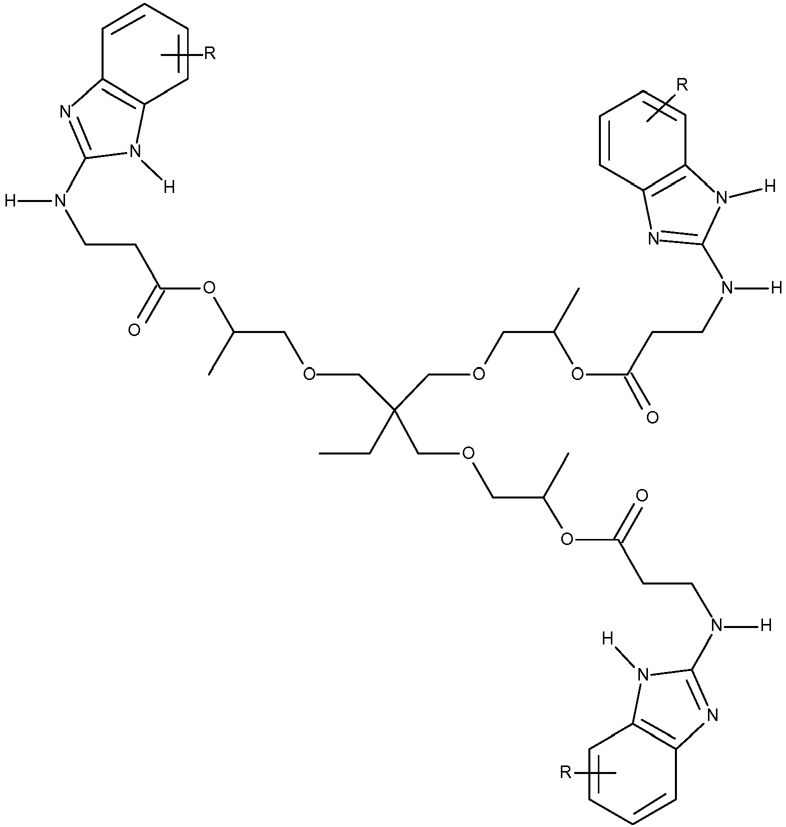 wobei für die hier gezeigten Strukturformeln **VII** bis **IX** gilt: **R** = gleich und/oder ungleich und = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl, wobei die Substituenten am Phenylrest wieder gleich oder ungleich und ausgewählt aus der folgenden Gruppe sein können: **R_{phenyl}** = Alkyl (linear und verzweigt), Alkoxy, -F, -Cl, -Br, - J, Aldehyd, Keton, Säureester, Säureamid, Phosphonsäurederivat und/oder Sulfonsäurederivat.

12. Wickelbandisoliersystem nach einem der vorstehenden Ansprüche, wobei zumindest ein Bandbeschleuniger in Form einer Verbindung, deren Struktur abgeleitet ist von zumindest einer der folgenden Grundstrukturen **X** bis **XII**, enthalten ist: 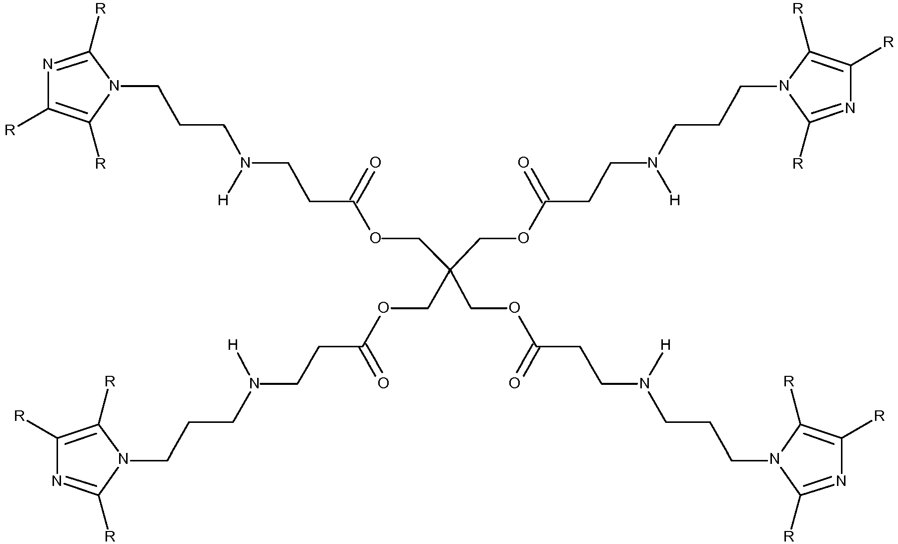 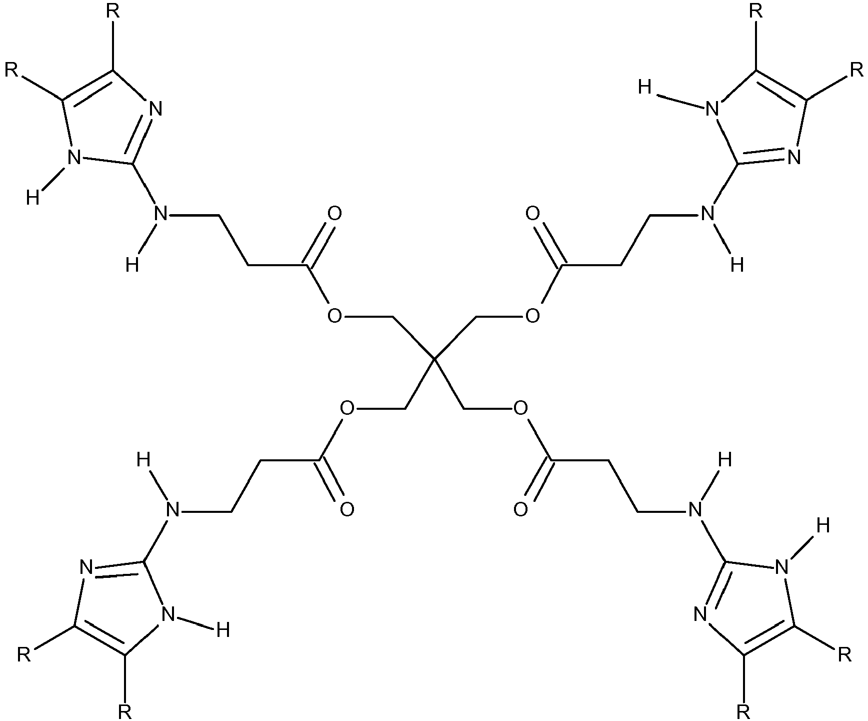 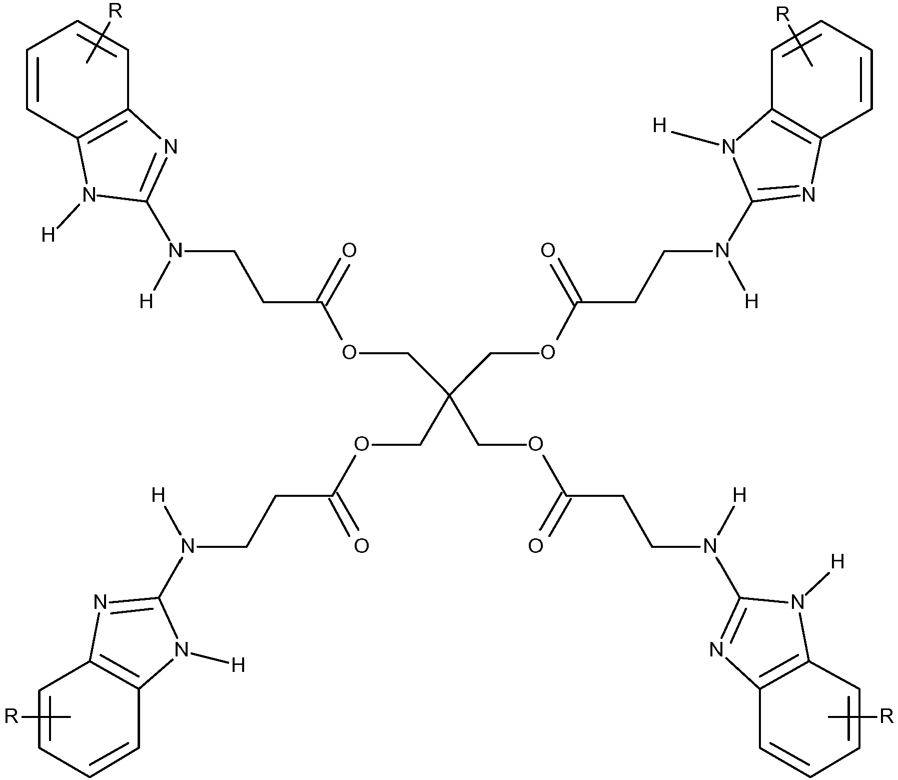 wobei für die hier gezeigten Strukturformeln **X** bis **XII** gilt: **R** = gleich und/oder ungleich und = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl, wobei die Substituenten am Phenylrest wieder gleich oder ungleich und ausgewählt aus der folgenden Gruppe sein können: **R_{phenyl}** = Alkyl (linear und verzweigt), Alkoxy, -F, -Cl, -Br, - J, Aldehyd, Keton, Säureester, Säureamid, Phosphonsäurederivat und/oder Sulfonsäurederivat.

13. Wickelbandisoliersystem nach einem der vorstehenden Ansprüche, wobei zumindest ein Bandbeschleuniger in Form einer Verbindung, deren Struktur abgeleitet ist von der folgenden Grundstruktur **XIII**, enthalten ist: 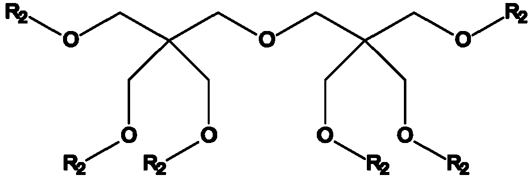 wobei
R₂ = H und/oder 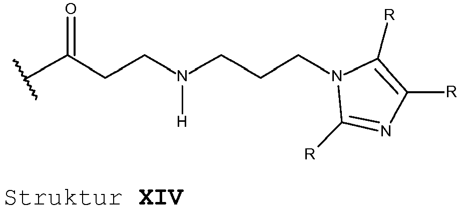 und/oder 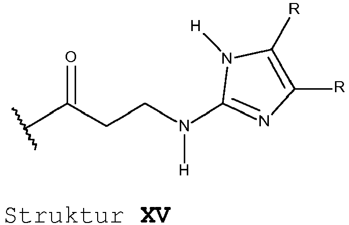 und/oder 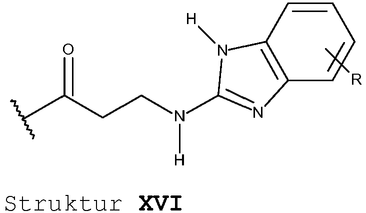 Für die gezeigte Grundstruktur **XIII** gilt:
**R** = gleich und/oder ungleich und
**R** = H, Methyl, Ethyl, n-Propyl, iso-Propyl, n-Butyl, iso-Butyl, tert-Butyl, Phenyl und/oder mono-, di-, tri-, tetra-, pentasubstituiertes Phenyl,
wobei die Substituenten am Phenylrest wieder gleich oder ungleich und ausgewählt aus der folgenden Gruppe sein können: **R_{phenyl}** = Alkyl (linear und verzweigt), Alkoxy, F, Cl, Br, J, Aldehyd, Keton, Säureester, Säureamid, Phosphonsäurederivat und/oder Sulfonsäurederivat
**R₂** wie in den Strukturen **XIV** bis **XVI** angegeben.

14. Verwendung eines Wickelbandisoliersystems nach einem der vorstehenden Ansprüche in Mittel- und Hochspannungsmaschinen, insbesondere rotierenden elektrischen Maschinen im Mittel- und Hochspannungsbereich sowie in elektrischen Schaltanlagen, Mittel- und Hochspannungsanwendungen, Durchführungen, Transformatordurchführungen, Generatordurchführungen und/oder HGÜ-Durchführungen, sowie in entsprechenden Halbzeugen.

15. Elektrische Maschine, bevorzugt rotierende elektrische Maschine, besonders bevorzugt rotierende elektrische Maschine im Mittel- und Hochspannungsbereich sowie elektrische Schaltanlage, Mittel- und Hochspannungsanwendung, Durchführung, Transformatordurchführung, Generatordurchführung und/oder HGÜ-Durchführung, sowie entsprechendes Halbzeug, die eine Isolation, hergestellt aus einem Wickelbandisoliersystem nach einem der Ansprüche 1 bis 12, umfasst.

## Claims

1. Wrapping-tape insulating system comprising an insulating tape with at least one tape adhesive, tape accelerator ultrafinely divided therein, and an anhydride-free impregnating resin, wherein there is at least one tape accelerator based on an amino-imidazole and/or aminoalkyl-imidazole and/or any desired derivatives thereof, bonded via the amino function to an acrylate by aza-Michael addition, wherein the wrapping-tape insulating system is producible by vacuum pressure impregnation.

2. Wrapping-tape insulating system according to Claim 1, wherein at least one amino-imidazole is selected from the group of the following imidazoles: aminoalkyl-imidazoles generally, preferably 1-(aminoalkyl)imidazole, 1H-2-aminoimidazole, 1H-2-(aminoalkyl)imidazole, 1H-4-aminoimidazole, 1H-4-(aminoalkyl)imidazole, 1H-5-aminoimidazole, 1H-5-(aminoalkyl)-imidazole, and also the corresponding derivatives 1-(3-aminopropyl)imidazole (CAS No. 5036-48-6),
1H-2-aminoimidazoles (CAS No. 7720-39-0),
1H-2-aminomethylimidazole
1H-2-aminoethylimidazole
1H-2-aminopropylimidazole
1H-2-aminobenzimidazoles (CAS No. 934-32-7).

3. Wrapping-tape insulating system according to Claim 1 or 2, wherein at least one amino-imidazole is selected from the group of the imidazoles which can be derived from the following structures **I** and **II:** 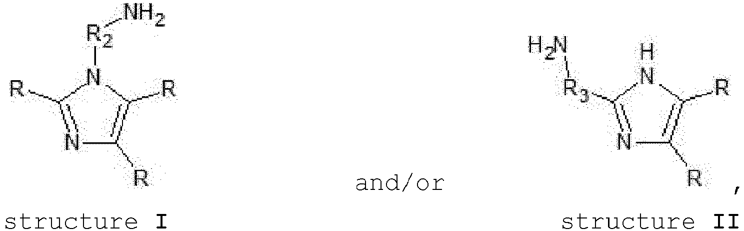 where
R₂ = (-CH₂-)ₙ wherein n = 1, 2, 3, ..., 10,
R₃ = (-CH₂-)ₙ wherein n = 1, 2, 3, ..., 10,
and R (identical and/or nonidentical) = methyl, ethyl, n-propyl, isopropyl, n-butyl, isobutyl, tert-butyl, aryl, more particularly phenyl.

4. Wrapping-tape insulating system according to any of the preceding Claims 1 to 3, wherein at least one acrylate is a higher acrylate and more particularly is selected from the group of the following acrylates: diacrylates, triacrylates, tetra-, penta- and/or hexaacrylates.

5. Wrapping-tape insulating system according to any of preceding Claims 1 to 4, wherein at least one higher acrylate is selected from the group of the following acrylates:
trimethylolpropane triacrylate (TMPTA, CAS No. 15625-89-5), trimethylolpropane propoxylate triacrylate (CAS No. 53879-54-2), pentaerythritol tetraacrylate (PETA, CAS No. 4986-89-4), dipentaerythritol pentaacrylate/dipentaerythritol hexaacrylate (CAS No. 60506-81-2) .

6. Wrapping-tape insulating system according to any of the preceding claims, wherein there is at least one tape adhesive in the form of a copolyester, diol and/or higher alcohol, and also any desired mixtures thereof.

7. Wrapping-tape insulating system according to any of the preceding claims, wherein the tape accelerator is present in the insulating tape in an amount of less than 10 wt%, based on the amount of impregnating resin.

8. Wrapping-tape insulating system according to any of the preceding claims, wherein the tape adhesive is present in the insulating tape in an amount in the range from 1 to 30 wt%, based on the amount of impregnating resin.

9. Wrapping-tape insulating system according to any of the preceding claims, wherein at least one tape accelerator is in the form of a compound having the following structure **III.**

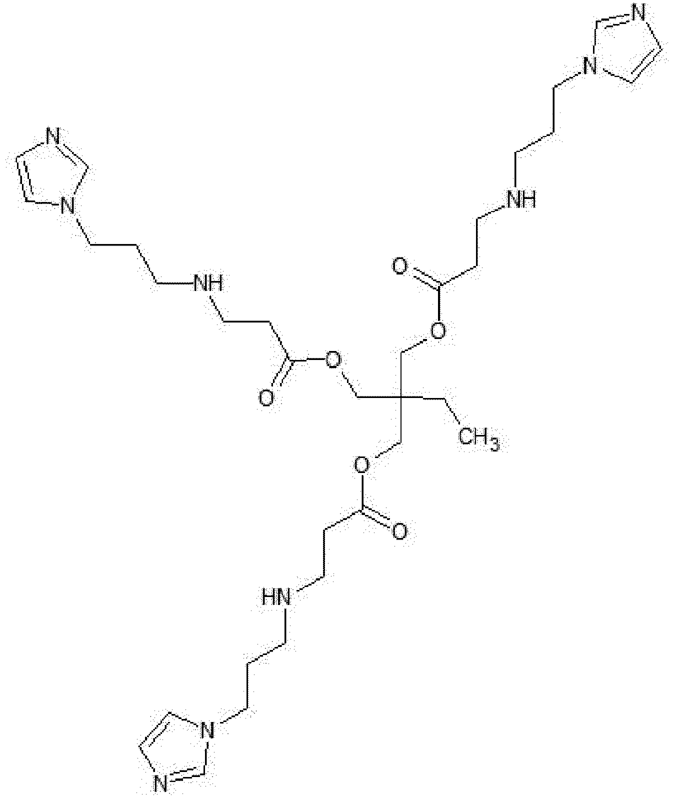

10. Wrapping-tape insulating system according to any of the preceding claims, wherein there is at least one tape accelerator in the form of a compound whose structure is derived from at least one of the following parent structures **IV** to **VI:** 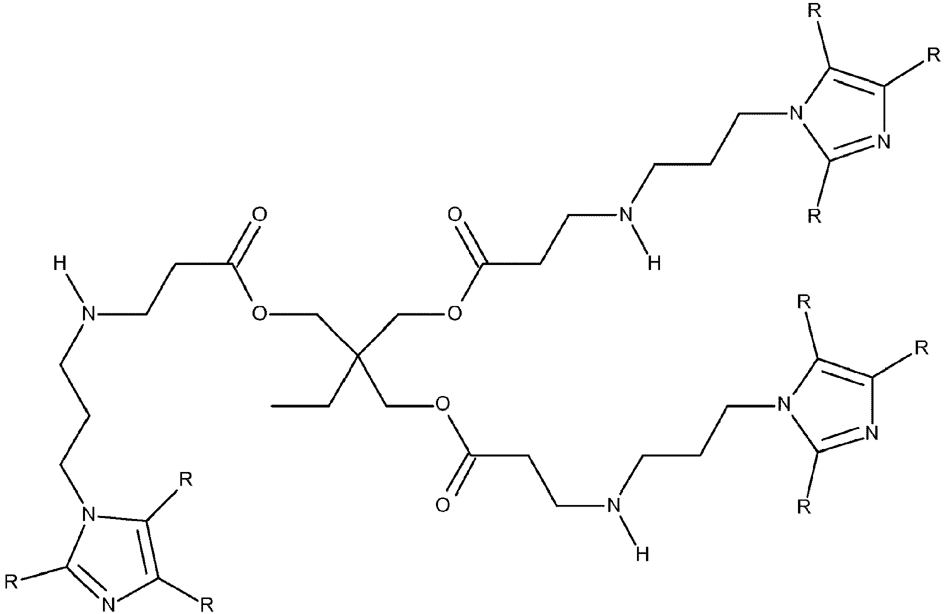 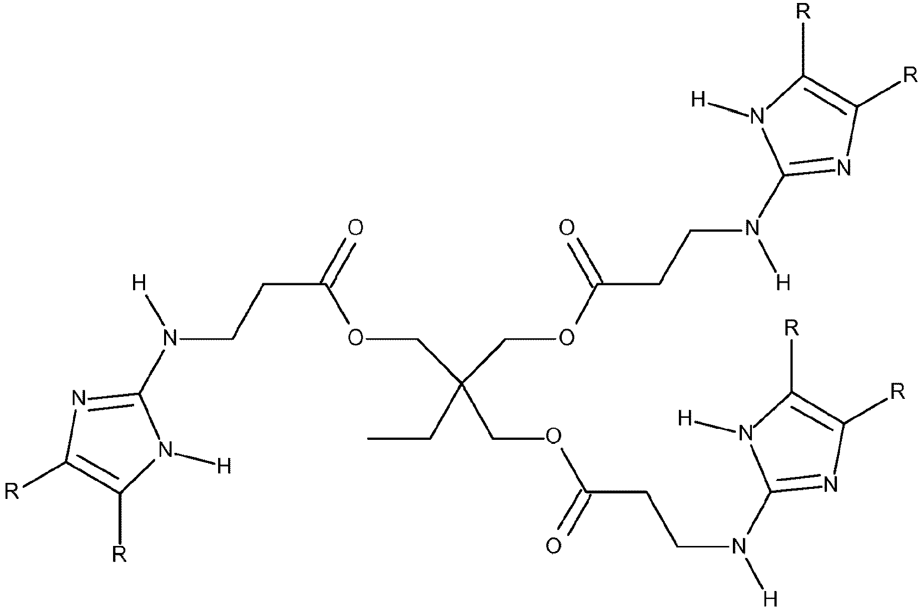 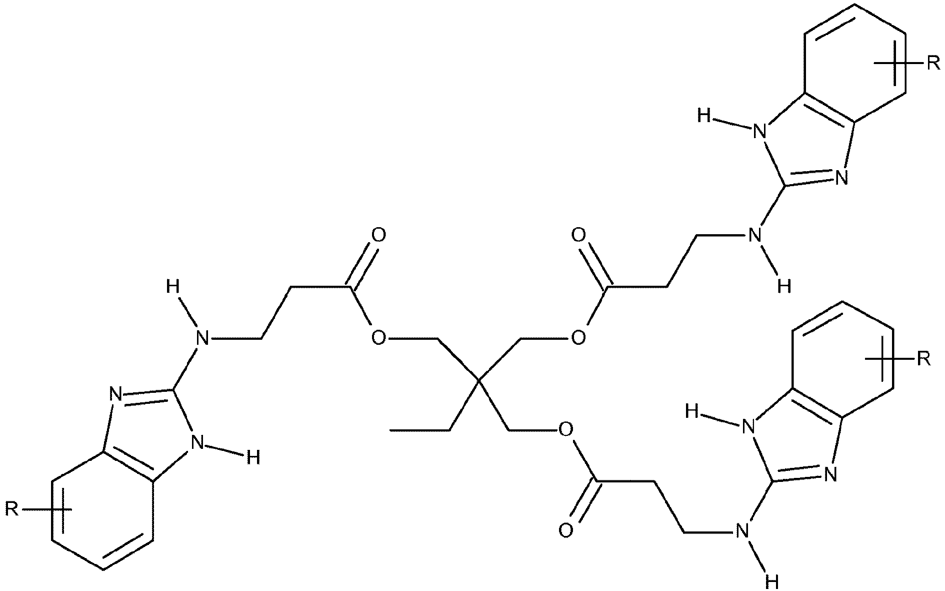 wherein, for the structural formulae **IV** to **VI** shown here:
**R** = identical and/or nonidentical and = H, methyl, ethyl, n-propyl, isopropyl, n-butyl, isobutyl, tert-butyl, phenyl and/or mono-, di-, tri-, tetra-, pentasubstituted phenyl, wherein the substituents on the phenyl radical may in turn be identical or nonidentical and may have been selected from the following group:
**R_{phenyl}** = alkyl (linear and branched), alkoxy, -F, -Cl, -Br, -I, aldehyde, ketone, acyl ester, acyl amide, phosphonic acid derivative and/or sulfonic acid derivative.

11. Wrapping-tape insulating system according to any of the preceding claims, wherein there is at least one tape accelerator in the form of a compound whose structure is derived from at least one of the following parent structures **VII** to **IX:** 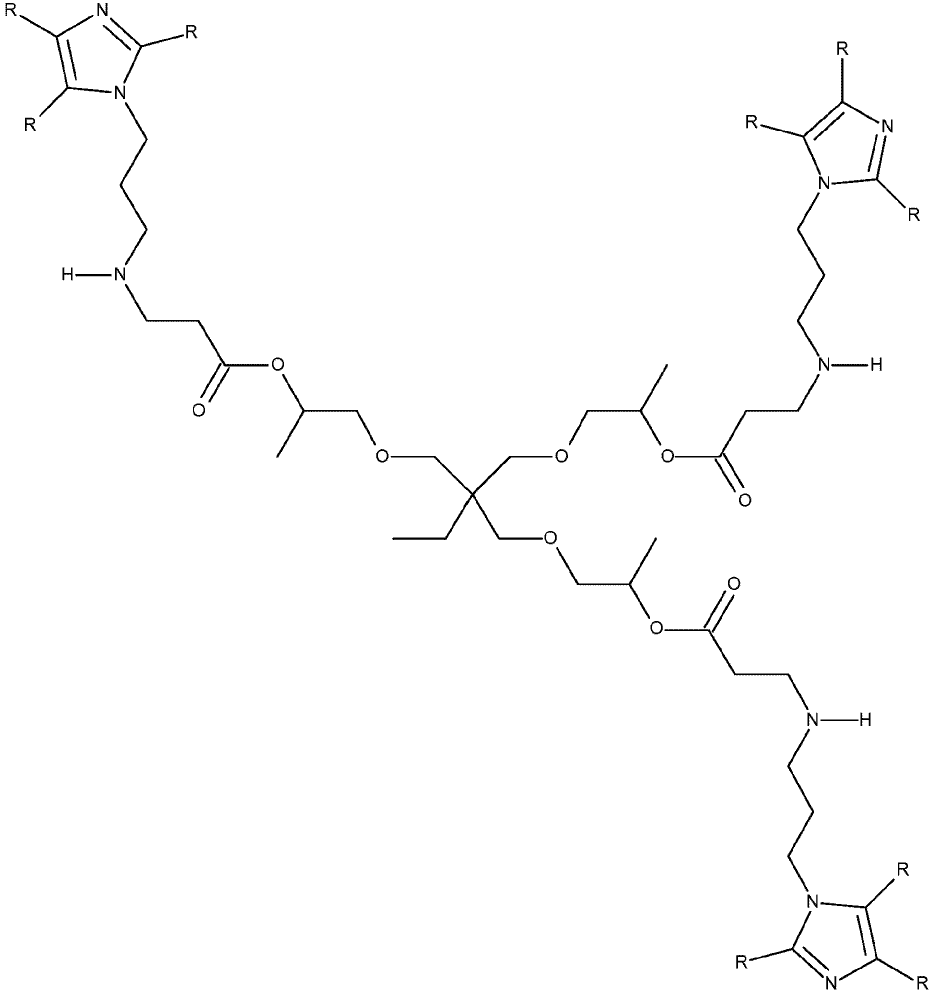 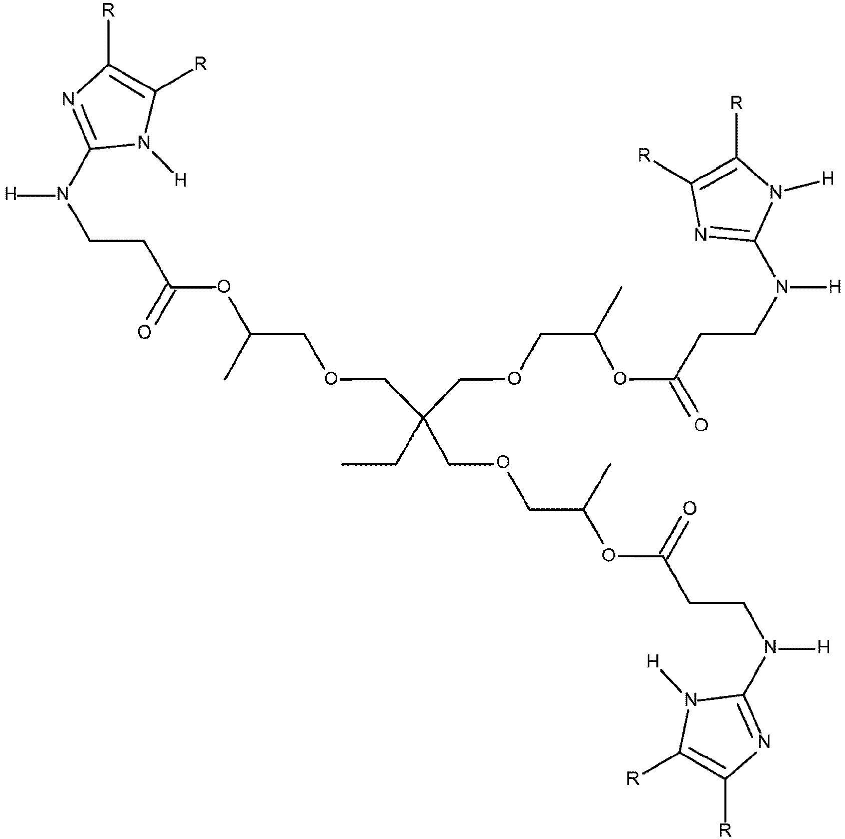 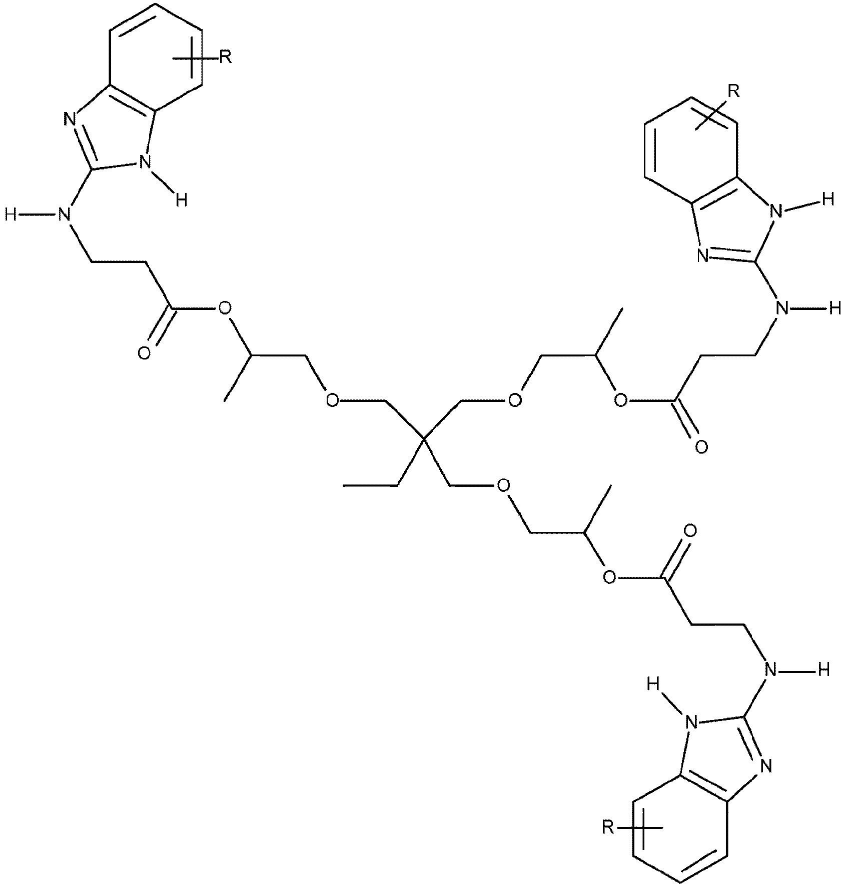 wherein, for the structural formulae **VII** to **IX** shown here:
**R** = identical and/or nonidentical and = H, methyl, ethyl, n-propyl, isopropyl, n-butyl, isobutyl, tert-butyl, phenyl and/or mono-, di-, tri-, tetra-, pentasubstituted phenyl, wherein the substituents on the phenyl radical may in turn be identical or nonidentical and may have been selected from the following group: **R_{phenyl}** = alkyl (linear and branched), alkoxy, -F, -Cl, -Br, -I, aldehyde, ketone, acyl ester, acyl amide, phosphonic acid derivative and/or sulfonic acid derivative.

12. Wrapping-tape insulating system according to any of the preceding claims, wherein there is at least one tape accelerator in the form of a compound whose structure is derived from at least one of the following parent structures **X** to **XII:** 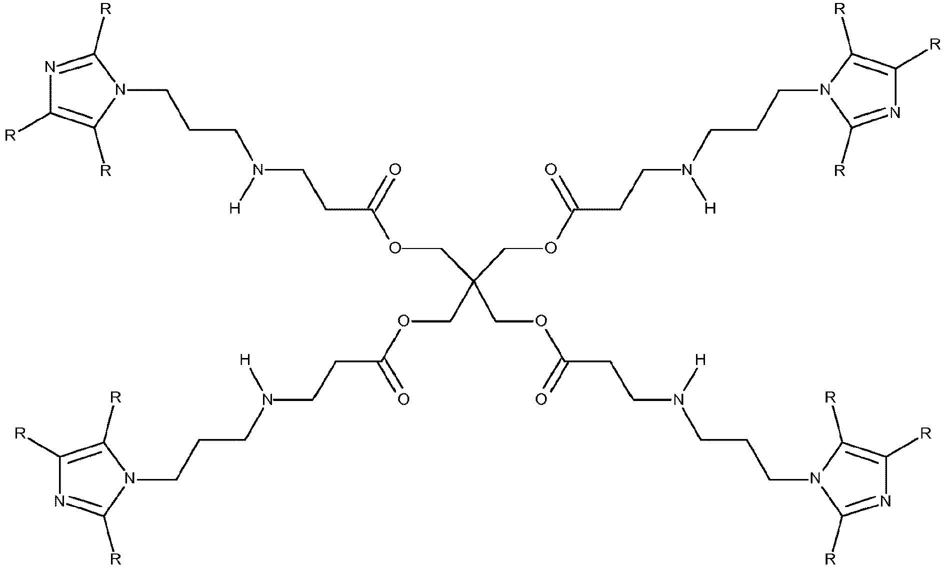 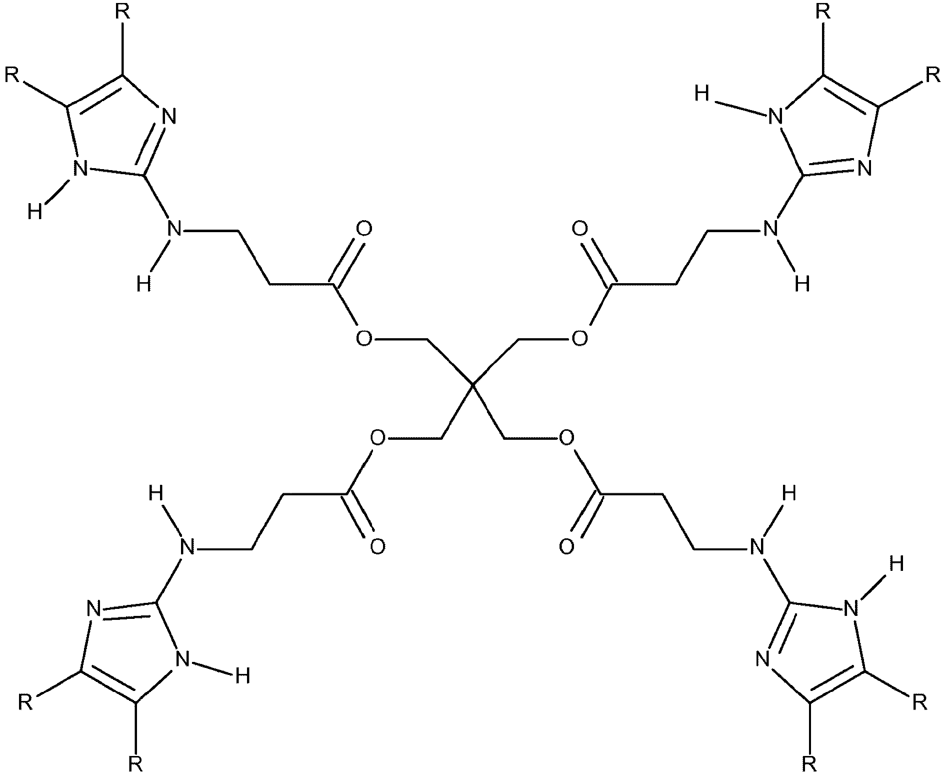 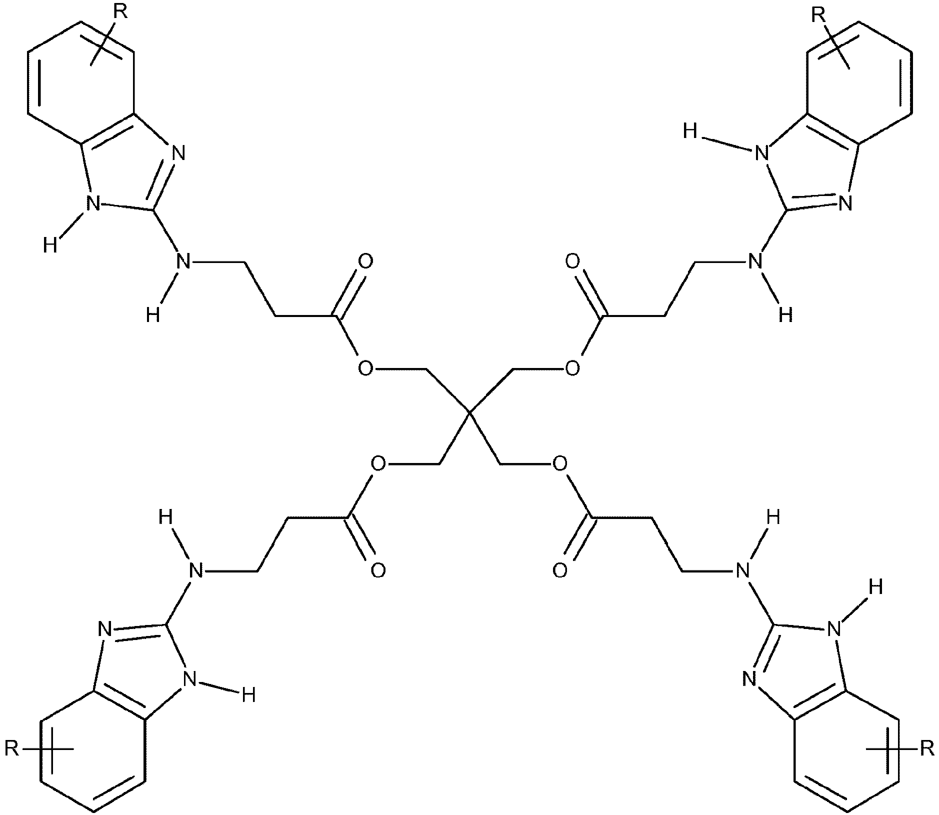 wherein, for the structural formulae **X** to **XII** shown here:
**R** = identical and/or nonidentical and = H, methyl, ethyl, n-propyl, isopropyl, n-butyl, isobutyl, tert-butyl, phenyl and/or mono-, di-, tri-, tetra-, pentasubstituted phenyl, wherein the substituents on the phenyl radical may in turn be identical or nonidentical and may have been selected from the following group:
**R_{phenyl}** = alkyl (linear and branched), alkoxy, -F, -Cl, -Br, -I, aldehyde, ketone, acyl ester, acyl amide, phosphonic acid derivative and/or sulfonic acid derivative.

13. Wrapping-tape insulating system according to any of the preceding claims, wherein there is at least one tape accelerator in the form of a compound whose structure is derived from the following parent structure **XIII**: 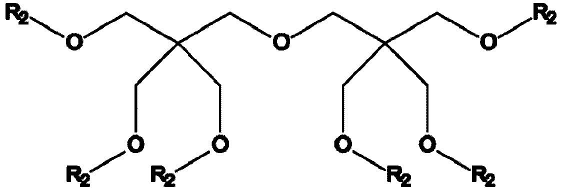 wherein
R₂ = H and/or 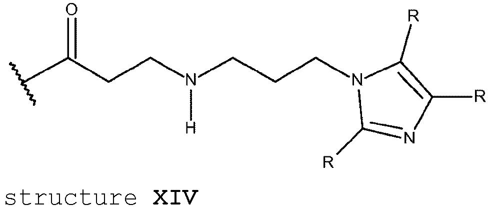 and/or 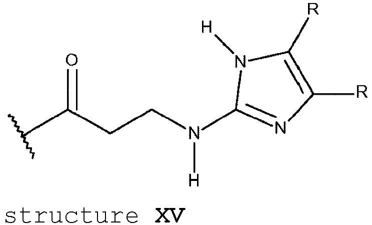 and/or 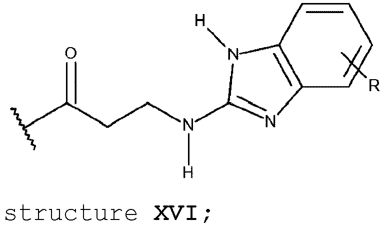 for the parent structure **XIII** shown:
**R** = identical and/or nonidentical and
**R** = H, methyl, ethyl, n-propyl, isopropyl, n-butyl, isobutyl, tert-butyl, phenyl and/or mono-, di-, tri-, tetra-, pentasubstituted phenyl,
wherein the substituents on the phenyl radical may in turn be identical or nonidentical and may have been selected from the following group:
**R_{phenyl}** = alkyl (linear and branched), alkoxy, F, Cl, Br, I, aldehyde, ketone, acyl ester, acyl amide, phosphonic acid derivative and/or sulfonic acid derivative
**R₂** as indicated in the structures **XIV** to **XVI.**

14. Use of a wrapping-tape insulating system according to any of the preceding claims in medium-voltage and high-voltage machines, more particularly rotating electrical machines in the medium-voltage and high-voltage range, and also in electrical switchgear, medium-voltage and high-voltage applications, bushings, transformer bushings, generator bushings and/or HVDC bushings, and also in corresponding semifinished products.

15. Electrical machine, preferably rotating electrical machine, more preferably rotating electrical machine in the medium-voltage and high-voltage range, and also electrical switchgear, medium-voltage and high-voltage application, bushing, transformer bushing, generator bushing and/or HVDC bushing, and also corresponding semifinished product, which comprises insulation produced from a wrapping-tape insulating system according to any of Claims 1 to 12.

## Revendications

1. Système d'isolation sous la forme d'un ruban à enrouler, qui comprend un ruban d'isolation comprenant au moins un adhésif destiné à un ruban dans lequel est incorporé, à l'état très finement dispersé, un accélérateur destiné à un ruban, ainsi qu'une résine d'imprégnation exempte d'anhydride ; dans lequel est présent au moins un accélérateur destiné à un ruban, à base d'un amino-imidazole et/ou d'un aminoalkyl-imidazole et/ou de n'importe lequel de leurs dérivés relié à un acrylate par l'intermédiaire d'une addition d'aza-Michael en passant par la fonction amino ; dans lequel le système d'isolation sous la forme d'un ruban à enrouler peut être obtenu à l'intervention d'une imprégnation par vide et pression.

2. Système d'isolation sous la forme d'un ruban à enrouler selon la revendication 1, dans lequel au moins un amino-imidazole est choisi parmi le groupe des imidazoles repris ci-après : des aminoalkyl-imidazoles en général, de préférence le 1-(aminoalkyl)-imidazole, le 1H-2-amino-imidazole, le 1H-2-(aminoalkyl)-imidazole, le 1H-4-amino-imidazole, le 1H-4-(aminoalkyl)-imidazole, le 1H-5-amino-imidazole, le 1H-5-(aminoalkyl)-imidazole, de même que les dérivés correspondants, à savoir :
le 1-(3-aminopropyl)-imidazole (numéro CAS 5036-48-6) ;
les 1H-2-aminoimidazoles (numéro CAS 7720-39-0) ;
le 1H-2-aminométhylimidazole ;
le 1H-2-aminoéthylimidazole ;
le 1H-2-aminopropylimidazole ;
les 1H-2-aminobenzimidazoles (numéro CAS 934-32-7).

3. Système d'isolation sous la forme d'un ruban à enrouler selon la revendication 1 ou 2, dans lequel au moins un amino-imidazole est choisi parmi le groupe des imidazoles que l'on peut dériver à partir des structures I et II indiquées ci-après : 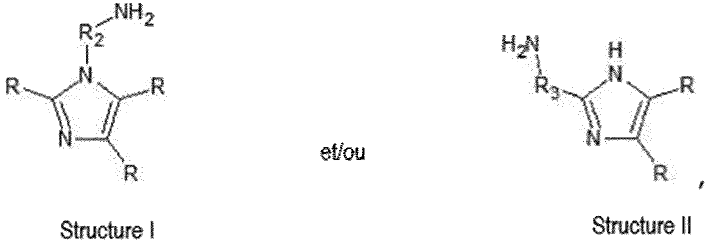 dans lesquelles :
R₂ représente un groupe (-CH₂-)ₙ dans lequel n est égal à 1, 2, 3, .., 10 ;
R₃ représente un groupe (-CH₂-)ₙ dans lequel n est égal à 1, 2, 3, ..., 10 ; et
R représente (de manière identique et/ou différente) : un groupe méthyle, un groupe éthyle, un groupe n-propyle, un groupe iso-propyle, un groupe n-butyle, un groupe iso-butyle, un groupe tert-butyle, un groupe aryle, en particulier un groupe phényle.

4. Système d'isolation sous la forme d'un ruban à enrouler selon l'une quelconque des revendications précédentes 1 à 3, dans lequel au moins un acrylate représente un acrylate supérieur et est choisi en particulier parmi le groupe des acrylates indiqués ci-après : des diacrylates, des triacrylates, des tétra-, penta- et/ou hexa-acrylates.

5. Système d'isolation sous la forme d'un ruban à enrouler selon l'une quelconque des revendications précédentes 1 à 4, dans lequel au moins un acrylate supérieur est choisi parmi le groupe des acrylates indiqués ci-après : le triacrylate de triméthylolpropane (TMPTA, numéro CAS 15625-89-5), le propoxytriacrylate de triméthylolpropane (numéro CAS 53879-54-2), le tétraacrylate de pentaérythritol (PETA, numéro CAS 4986-89-4), le pentaacrylate de dipentaérythritol/hexaacrylate de dipentaérythritol (numéro CAS 60506-81-2).

6. Système d'isolation sous la forme d'un ruban à enrouler selon l'une quelconque des revendications précédentes, dans lequel est présent au moins un adhésif destiné à un ruban sous la forme d'un copolyester, d'un diol et/ou d'un alcool supérieur, ainsi que sous la forme de l'un quelconque de leurs mélanges.

7. Système d'isolation sous la forme d'un ruban à enrouler selon l'une quelconque des revendications précédentes, dans lequel l'accélérateur destiné à un ruban est présent dans le ruban d'isolation en une quantité qui est inférieure à 10 % en poids, rapportés à la quantité de la résine d'imprégnation.

8. Système d'isolation sous la forme d'un ruban à enrouler selon l'une quelconque des revendications précédentes, dans lequel l'adhésif destiné à un ruban est présent dans le ruban d'isolation en une quantité qui se situe dans la plage de 1 à 30 % en poids, rapportés à la quantité de la résine d'imprégnation.

9. Système d'isolation sous la forme d'un ruban à enrouler selon l'une quelconque des revendications précédentes, dans lequel est présent au moins un accélérateur destiné à un ruban, sous la forme d'un composé qui possède la structure III indiquée ci-après.

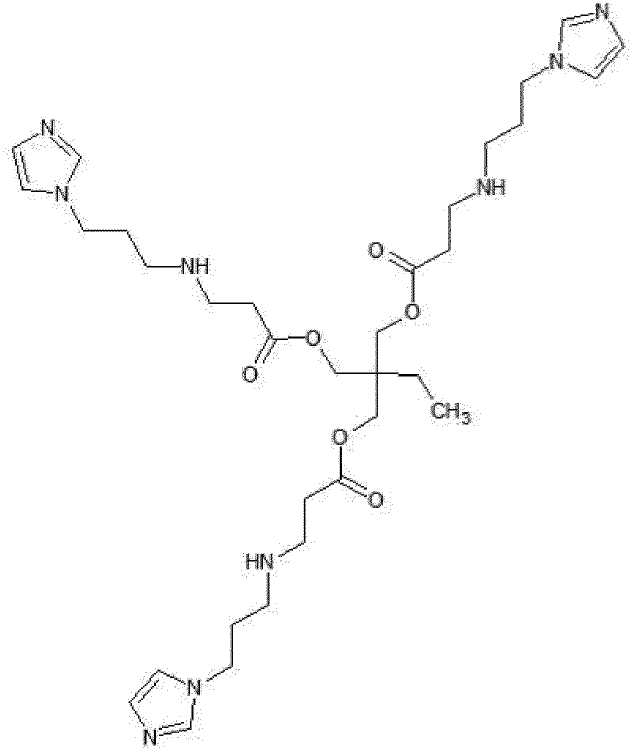

10. Système d'isolation sous la forme d'un ruban à enrouler selon l'une quelconque des revendications précédentes, dans lequel est contenu au moins un accélérateur destiné à un ruban sous la forme d'un composé dont la structure dérive d'au moins une des structures de base IV à VI indiquées ci-après : 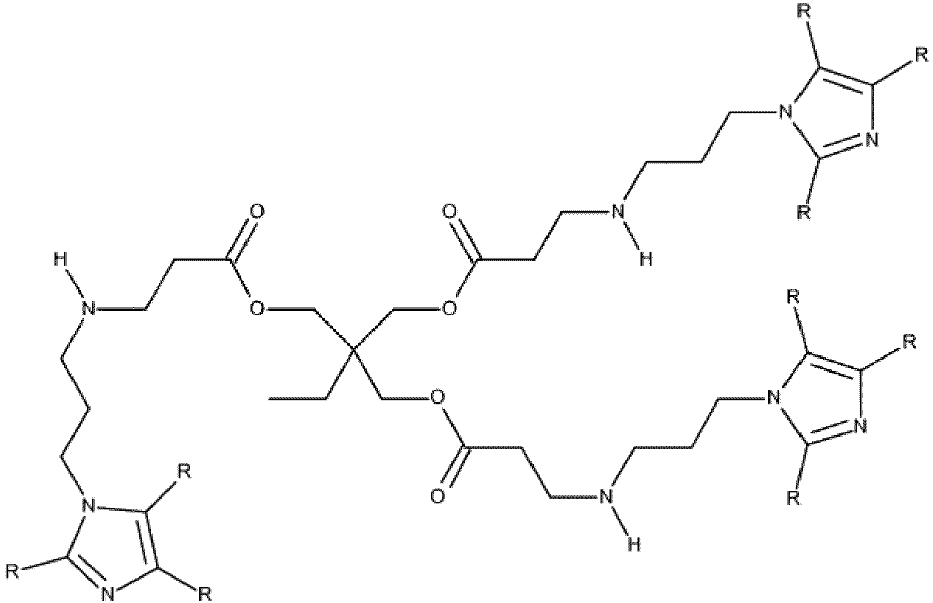 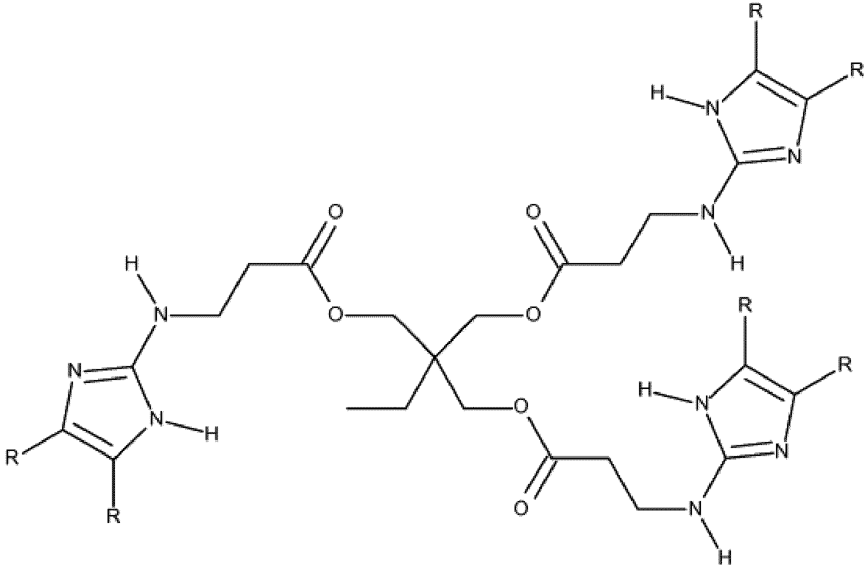 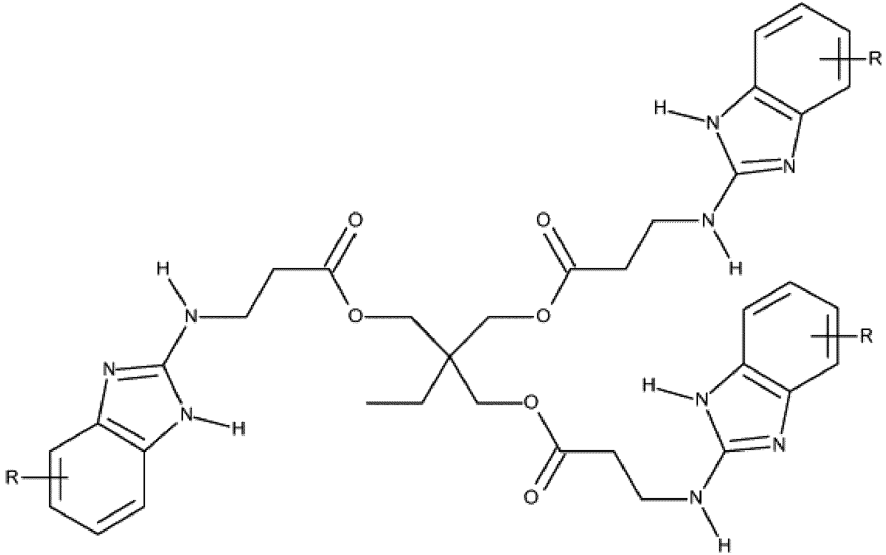 dans lesquelles, pour les formules développées IV à VI indiquées en l'occurrence :
R concerne des radicaux identiques et/ou différents et représente un atome d'hydrogène, un groupe méthyle, un groupe éthyle, un groupe n-propyle, un groupe iso-propyle, un groupe n-butyle, un groupe iso-butyle, un groupe tert-butyle, un groupe phényle et/ou un groupe phényle mono-, di-, tri-, tétra-, pentasubstitué ; dans lequel les substituants sur le radical phényle peuvent être une nouvelle fois identiques ou différents et peuvent être choisis parmi le groupe indiqué ci-après :
R_{phényle} représente un groupe alkyle (à chaîne droite et à chaîne ramifiée), un groupe alcoxy, un atome de fluor, un atome de chlore, un atome de brome, un atome d'iode, un groupe aldéhyde, un groupe cétone, un groupe ester d'acide, un groupe amide d'acide, un dérivé de l'acide phosphonique et/ou un dérivé de l'acide sulfonique.

11. Système d'isolation sous la forme d'un ruban à enrouler selon l'une quelconque des revendications précédentes, dans lequel est contenu au moins un accélérateur destiné à un ruban sous la forme d'un composé dont la structure dérive d'au moins une des structures de base VII à IX indiquées ci-après : 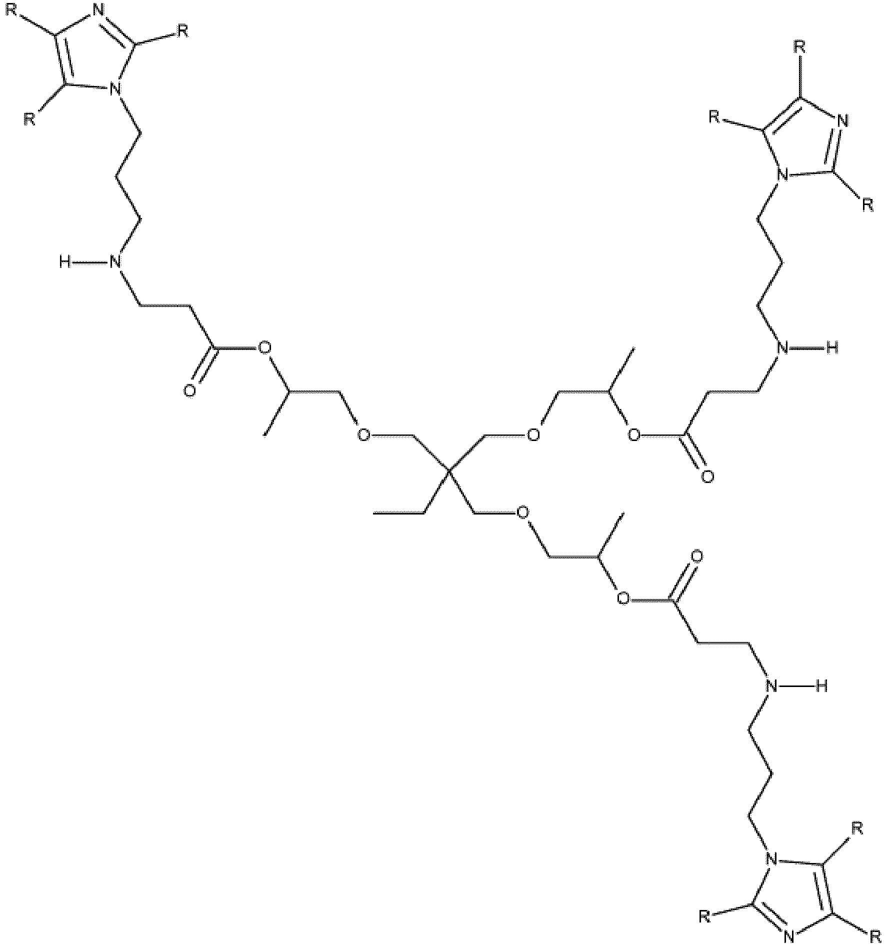 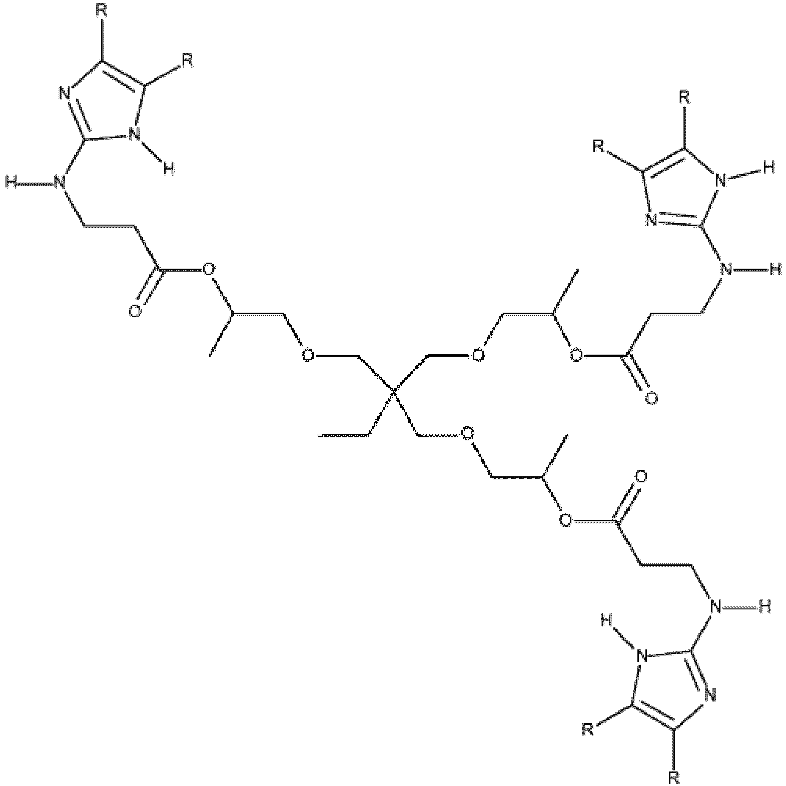 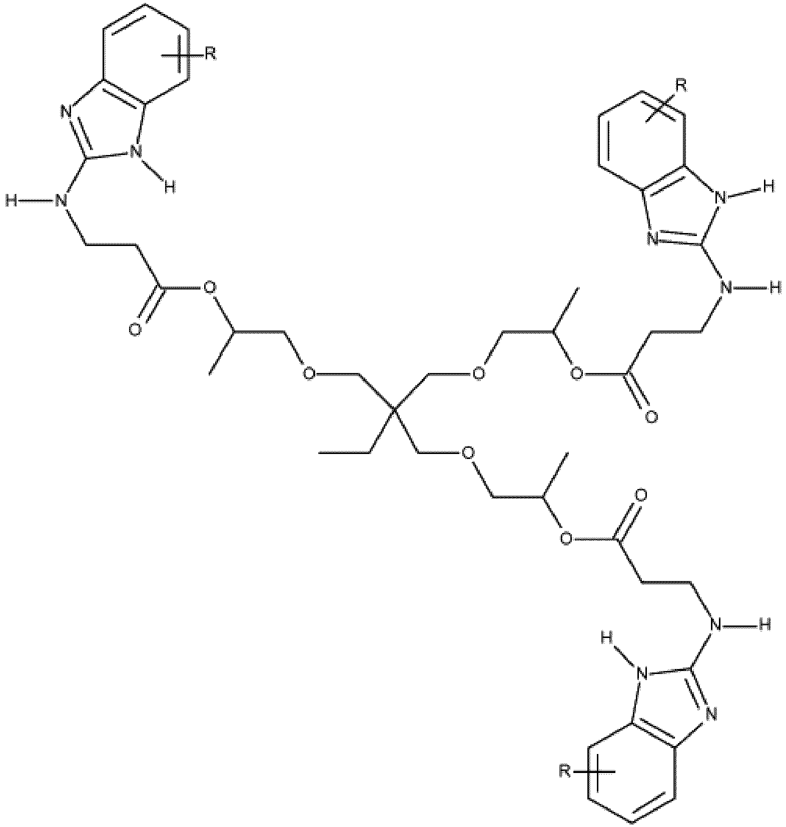 dans lesquelles, pour les formules développées VII à IX indiquées en l'occurrence :
R concerne des radicaux identiques et/ou différents et représente un atome d'hydrogène, un groupe méthyle, un groupe éthyle, un groupe n-propyle, un groupe iso-propyle, un groupe n-butyle, un groupe iso-butyle, un groupe tert-butyle, un groupe phényle et/ou un groupe phényle mono-, di-, tri-, tétra-, pentasubstitué ; dans lequel les substituants sur le radical phényle peuvent être une nouvelle fois identiques ou différents et peuvent être choisis parmi le groupe indiqué ci-après :
R_{phényle} représente un groupe alkyle (à chaîne droite et à chaîne ramifiée), un groupe alcoxy, un atome de fluor, un atome de chlore, un atome de brome, un atome d'iode, un groupe aldéhyde, un groupe cétone, un groupe ester d'acide, un groupe amide d'acide, un dérivé de l'acide phosphonique et/ou un dérivé de l'acide sulfonique.

12. Système d'isolation sous la forme d'un ruban à enrouler selon l'une quelconque des revendications précédentes, dans lequel est contenu au moins un accélérateur destiné à un ruban sous la forme d'un composé dont la structure dérive d'au moins une des structures de base X à XII indiquées ci-après : 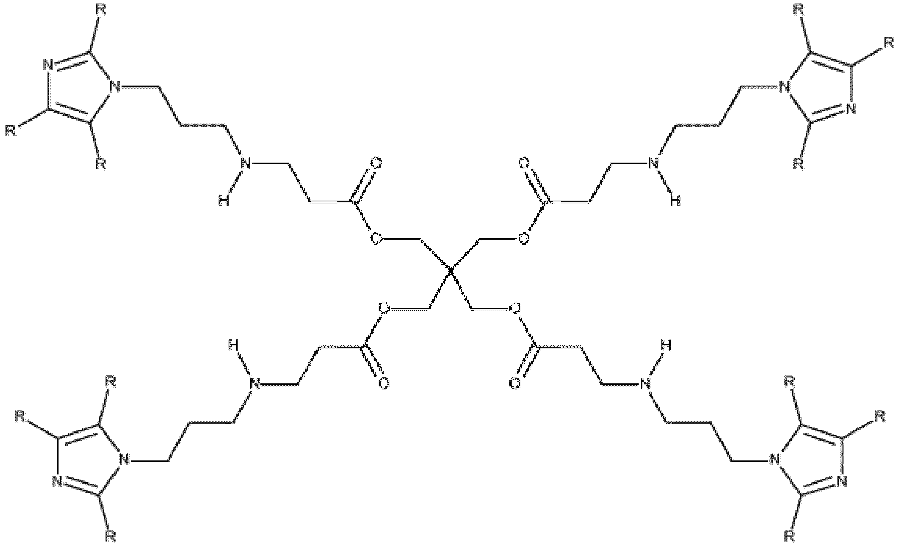 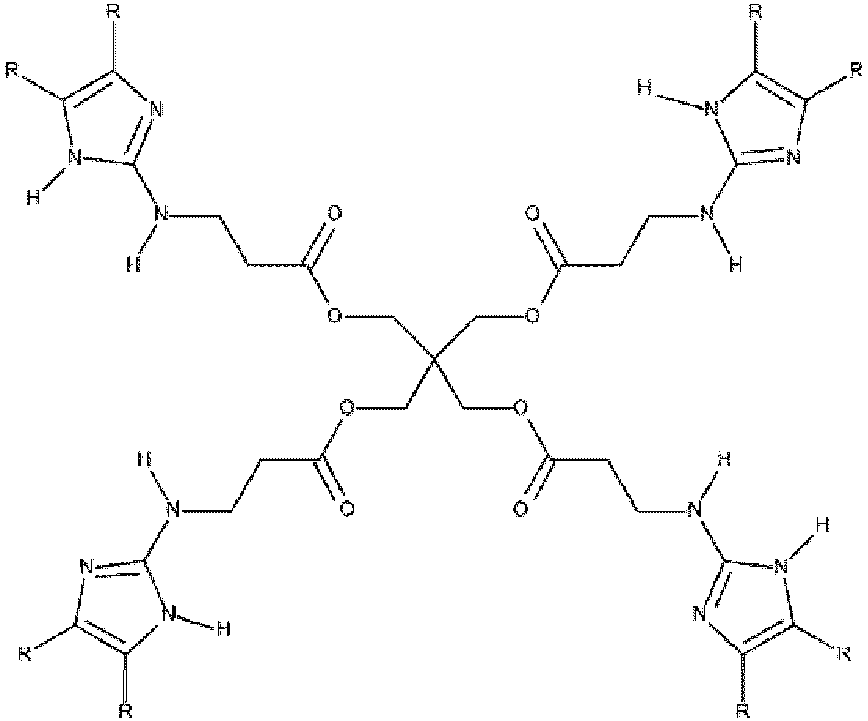 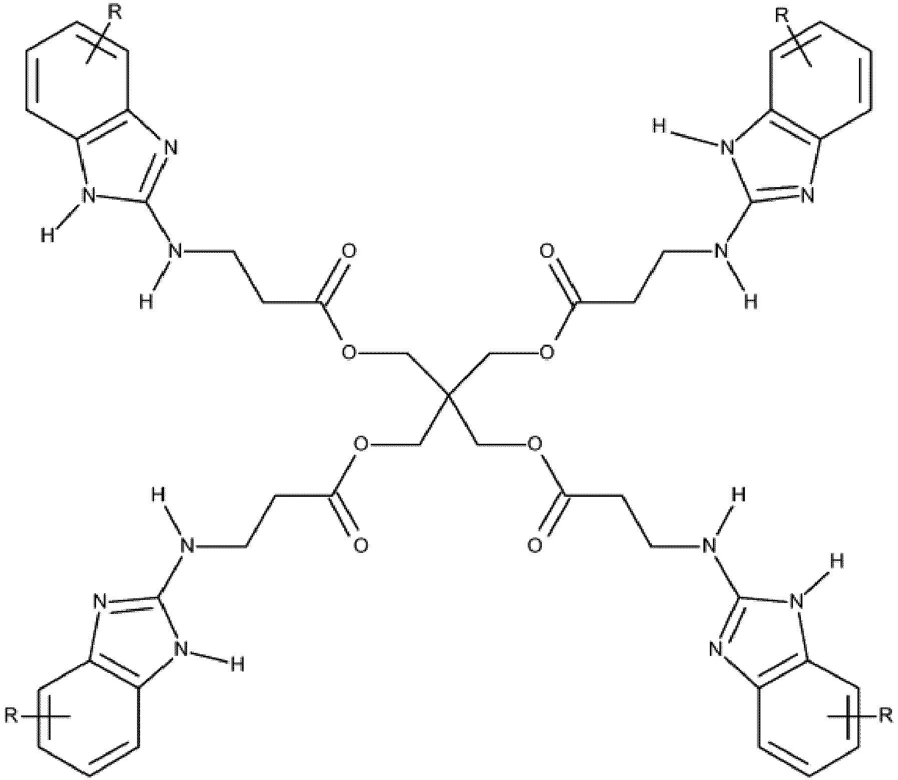 dans lesquelles, pour les formules développées X à XII indiquées en l'occurrence :
R concerne des radicaux identiques et/ou différents et représente un atome d'hydrogène, un groupe méthyle, un groupe éthyle, un groupe n-propyle, un groupe iso-propyle, un groupe n-butyle, un groupe iso-butyle, un groupe tert-butyle, un groupe phényle et/ou un groupe phényle mono-, di-, tri-, tétra-, pentasubstitué ; dans lequel les substituants sur le radical phényle peuvent être une nouvelle fois identiques ou différents et peuvent être choisis parmi le groupe indiqué ci-après :
R_{phényle} représente un groupe alkyle (à chaîne droite et à chaîne ramifiée), un groupe alcoxy, un atome de fluor, un atome de chlore, un atome de brome, un atome d'iode, un groupe aldéhyde, un groupe cétone, un groupe ester d'acide, un groupe amide d'acide, un dérivé de l'acide phosphonique et/ou un dérivé de l'acide sulfonique.

13. Système d'isolation sous la forme d'un ruban à enrouler selon l'une quelconque des revendications précédentes, dans lequel est contenu au moins un accélérateur destiné à un ruban sous la forme d'un composé dont la structure dérive de la structure de base XIII indiquée ci-après : 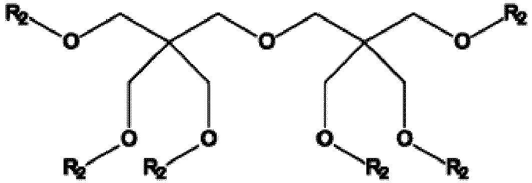 dans laquelle :
R₂ représente un atome d'hydrogène et/ou 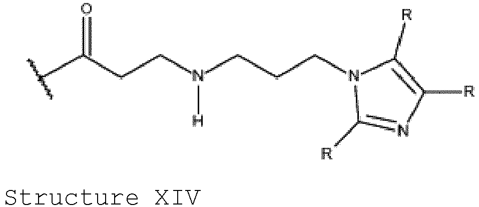 et/ou 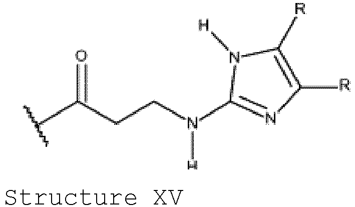 et/ou 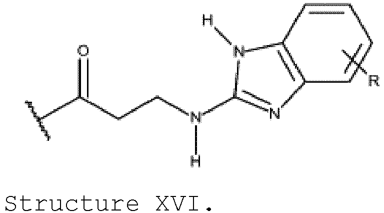 Pour la structure de base XIII indiquée :
R concerne des radicaux identiques et/ou différents ; et
R représente un atome d'hydrogène, un groupe méthyle, un groupe éthyle, un groupe n-propyle, un groupe iso-propyle, un groupe n-butyle, un groupe iso-butyle, un groupe tert-butyle, un groupe phényle et/ou un groupe phényle mono-, di-, tri-, tétra-, pentasubstitué ;
dans lequel les substituants sur le radical phényle peuvent être une nouvelle fois identiques ou différents et peuvent être choisis parmi le groupe indiqué ci-après :
R_{phényle} représente un groupe alkyle (à chaîne droite et à chaîne ramifiée), un groupe alcoxy, un atome de fluor, un atome de chlore, un atome de brome, un atome d'iode, un groupe aldéhyde, un groupe cétone, un groupe ester d'acide, un groupe amide d'acide, un dérivé de l'acide phosphonique et/ou un dérivé de l'acide sulfonique ;
R₂ est tel qu'indiqué dans les structures XIV à XVI.

14. Utilisation d'un système d'isolation sous la forme d'un ruban à enrouler selon l'une quelconque des revendications précédentes dans des machines travaillant dans des conditions de moyenne tension et de haute tension, en particulier des machines électriques rotatives dans le domaine de la moyenne tension et de la haute tension, ainsi que dans des installations de distribution électrique, des applications qui travaillent dans des conditions de moyenne tension et de haute tension, des traversées, des traversées de transformateurs, des traversées de générateurs et/ou des traversées pour la transmission d'un courant continu-haute tension, ainsi que dans des semi-conducteurs correspondants.

15. Machine électrique, de préférence machine électrique rotative, de manière particulièrement préférée machine électrique rotative dans le domaine de la moyenne tension et de la haute tension, et installation de distribution électrique, application qui travaille dans des conditions de moyenne tension et de haute tension, traversée, traversée de transformateur, traversée de générateur et/ou traversée de transmission de courant continu-haute tension, et semiconducteur correspondant, qui comprend une isolation que l'on obtient à partir d'un système d'isolation sous la forme d'un ruban à enrouler selon l'une quelconque des revendications 1 à 12.
